(19) Europäisches Patentamt / European Patent Office / Office européen des brevets

(11) **EP 4 478 894 B1**

(12) **EUROPEAN PATENT SPECIFICATION**

(45) Date of publication and mention
of the grant of the patent:
**12.11.2025 Bulletin 2025/46**

(21) Application number: **23706574.3**

(22) Date of filing: **17.02.2023**

(51) International Patent Classification (IPC):
*A23K 20/105* (2016.01)   *A23K 20/20* (2016.01)
*A23K 20/24* (2016.01)   *A23K 50/15* (2016.01)
*A23K 20/22* (2016.01)   *A23K 50/10* (2016.01)
*A23K 20/111* (2016.01)   *A23K 20/137* (2016.01)

(52) Cooperative Patent Classification (CPC):
**A23K 50/15; A23K 20/105; A23K 20/111;
A23K 20/137; A23K 20/22; A23K 20/24;
A23K 50/10;** Y02P 60/22

(86) International application number:
**PCT/EP2023/054086**

(87) International publication number:
**WO 2023/156621 (24.08.2023 Gazette 2023/34)**

(54) **A MIXTURE OF UREA, BIURET AND NITRATE AS A DIETARY NON-PROTEIN NITROGEN-SOURCE FOR RUMINANTS AND USES THEREOF**

MISCHUNG AUS HARNSTOFF, BIURET UND NITRAT ALS EINE ERNÄHRUNGSFREIE PROTEINFREIE STICKSTOFFQUELLE FÜR WIEDERKÄUER UND VERWENDUNGEN DAVON

MÉLANGE D'URÉE, DE BIURET ET DE NITRATE EN TANT QUE SOURCE D'AZOTE ALIMENTAIRE NON PROTÉIQUE POUR RUMINANTS ET UTILISATIONS ASSOCIÉES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **17.02.2022 EP 22157277
03.06.2022 EP 22382539
09.09.2022 EP 22382836**

(43) Date of publication of application:
**25.12.2024 Bulletin 2024/52**

(73) Proprietor: **Yara International ASA
0277 Oslo (NO)**

(72) Inventors:
• **RUIZ, Isabel
28806 Alcalá de Henares (Madrid) (ES)**
• **MARTINEZ-LUENGAS, Inés
28223 Pozuelo de Alarcón (Madrid) (ES)**
• **PIRRO, Laura
4541 HJ Terneuzen (NL)**
• **VAN BELZEN, Ruud
4541 HJ Terneuzen (NL)**

• **MOHAN, Anand
4541 HJ Terneuzen (NL)**
• **IPHARRAGUERRE, Ignacio, R.
08461 Barcelona (ES)**

(74) Representative: **De Clercq & Partners
Edgard Gevaertdreef 10a
9830 Sint-Martens-Latem (BE)**

(56) References cited:
CA-A- 1 241 663   CA-A- 976 189
LT-B- 6 282   US-A- 2 768 895
US-A- 3 878 304

• XIUMIN ZHANG ET AL: "Effects of urea plus nitrate pretreated rice straw and corn oil supplementation on fiber digestibility, nitrogen balance, rumen fermentation, microbiota and methane emissions in goats", JOURNAL OF ANIMAL SCIENCE AND BIOTECHNOLOGY, BIOMED CENTRAL LTD, LONDON, UK, vol. 10, no. 1, 23 January 2019 (2019-01-23), pages 1 - 10, XP021270875, DOI: 10.1186/S40104-019-0312-2

**(Cont. next page)**

- **VAN WYNGAARD J D V ET AL: "Effect of dietary nitrate on enteric methane emissions, production performance and rumen fermentation of dairy cows grazing kikuyu-dominant pasture during summer", ANIMAL FEED SCIENCE AND TECHNOLOGY, ELSEVIER, AMSTERDAM, NL, vol. 244, 13 August 2018 (2018-08-13), pages 76 - 87, XP085471303, ISSN: 0377-8401, DOI: 10.1016/ J.ANIFEEDSCI.2018.08.005**

## Description

### Technical field

The present disclosure relates to a ruminant feed supplement composition and to the field of dietary non-protein nitrogen (NPN)-sources. The present disclosure particularly relates to the use of such a ruminant feed supplement composition as a dietary non-protein nitrogen (NPN) source to improve nitrogen nutrition and to reduce methane emissions by ruminant animals.

### Background art

Ruminants are defined as large hoofed herbivorous grazing or browsing mammals that are able to acquire nutrients from plant-based feed by digesting it in a specialized stomach having four compartments, one of which is the rumen. Thanks to the microbial actions in the rumen prior to digestion, ruminants have the unique ability to use (low quality) protein sources and also NPN sources. This gives them a competitive advantage over other domesticated animals. The symbiotic relationship with the rumen microflora allows ruminants to upcycle nutrients to produce highly nutritious animal proteins, by means of an efficient pre-gastric fermentation of feeds, which takes place in the reticulorumen, the largest compartment of their gastrointestinal tract. Ruminants provide an optimum environment for microorganisms to thrive, and they, in turn, supply the ruminant host with microbial crude protein, which is of high quality in terms of amino acids supplement. In beef and dairy production systems, the microbial crude protein synthesized in the rumen provides the majority of the metabolizable protein required by the animal.

Increased utilization of N for bacterial synthesis and capture of dietary N into anabolic products, particularly absorbed amino acids, may be influenced by dietary manipulation, mainly by energy levels in the ration. However, there is an upper limit to the overall efficiency of the process: a maximum of 50 to 60% of dietary N, or 70 to 90% of apparent digested N will be converted into amino acids and released into the portal vein. For this reason, particularly in beef cattle diets, it is extremely important to assess the optimum kinetics of N release in NPN sources for each energy source (e.g., basal forage diet, supplemental by-products, etc.) to maximize N utilization and minimize excretion.

In most beef production systems, several grains and grain byproducts are available as supplemental protein sources. However, on a per kg of N supplied, it is nearly impossible to compete with the lower costs of supplemental crude protein in the form of NPN. Indeed, non-protein nitrogen, mainly in the form of urea, is known as a cost-effective source of supplemental nitrogen (N) in ruminant production. Commercial/feed-grade urea typically contains only traces of biuret, with a biuret content not exceeding 1.0 wt%. Urea is however usually not included in more than 1 wt.% of the diet. Any shortage of protein in a diet or supplementation program is frequently covered by the use of urea-based supplements in the form of range cubes, liquid or pelleted supplements and tubs. Apart from urea, other ammonia forming chemical compounds such as biuret and ammonium sulfate have been used in the formulation of ruminal diets to replace part of plant protein sources and providing adequate amounts of ruminal degradable protein for a better efficiency of fiber digestion and the synthesis of microbial protein. The use of NPN, however, has been traditionally limited by the fast rate of N-release in the rumen of a ruminant (animal) in the form of ammonia. If the release of ammonia in the rumen exceeds the use capacity by ruminal microbiota, this N-excess will be excreted, with a consequent loss of energy and N-efficiency. Furthermore, if the ammonia concentration overpasses the liver excretion capacity of the ruminant, ammonia toxicity or death may occur.

As a result, in the prior art, it has been aimed at improving the use of NPN to reduce the rate of N-release in the rumen. In recent years, several slow-release urea products have been developed and tested in ruminant nutrition, for instance by physical means, such as by applying a coating, with variable results. Applying a coating with the aim to obtain slow release means adding fat, wax or the like to the NPN-source. This has the disadvantage that the NPN-content is diluted. A coating furthermore increases the production cost of a dietary N-source in cattle diets that should be cost effective. Another solution is to apply chemical means such as using pure biuret. The disadvantage of pure biuret is that the production thereof is very expensive, because the heating time to obtain the pure biuret out of urea has to be extended a lot.

In addition, currently, there is growing interest in decreasing the environmental impact of ruminant production, including beef and dairy, and many strategies are being pursued to achieve this goal.

In the current ruminant feeding systems, methane ($CH_4$) production is an inevitable consequence of fermentative digestion. The microbial consortium of the rumen must dispose hydrogen that is produced during fermentation. Otherwise, hydrogen would accumulate in the rumen and thereby inhibit fermentation of ingested feeds. Therefore, the rumen microbial system is provided with methanogens that reduce carbon dioxide ($CO_2$) to methane.

The major hydrogen (electron) sink in the rumen is methane produced by the reduction of carbon dioxide using reduced co-enzymes such as NADH as the electron source. Methane is however an energy loss for ruminants and a potent greenhouse gas, which has a 28-times greater global warming potential than carbon dioxide.

Globally, ruminants produce some 80 million tons of methane annually, accounting for some 28% of manmade methane emissions. The adoption of ruminant management practices which result in major reductions of release of methane are consequently high priority.

It is already known that the supplementation of nitrate compounds may be a viable strategy to reduce methane emissions in ruminant production systems. However, the effects of nitrate compounds on high-forage diets have not been extensively studied, as well as potential combination of nitrate compounds with other NPN-sources. Most studies have been focusing on replacing urea almost completely with nitrates.

In WO 2017/181250, a controlled-release coated non-protein nitrogen food (feed) supplement for animal nutrition is described, particularly to be administered to ruminant animals, which is completely metabolized in the abomasum of the animal. This supplement comprises a core comprising 80 - 90 wt.% NPN-source and a multilayer coating surrounding the core which comprises at least four different layers. This patent application discloses a list of different components which could be used as an NPN-source in the core, preferably being urea (as the single component). However, no specific suitable combinations have been mentioned.

In the article with the title "Effects of urea plus nitrate pretreated rice straw and corn oil supplementation on fiber digestibility, nitrogen balance, rumen fermentation, microbiota and methane emissions in goats", Xiumin Zhang et al., Journal of Animal Science and Biotechnology, Biomed, Central Ltd., London, UK, vol. 10, no. 1, 23 January 2019, page 1 - 10, a study is described where the combined effects of urea plus nitrate pretreated rice straw and corn oil supplementation to the diet on nutrient digestibility, nitrogen (N) balance, $CH_4$ emissions, ruminal fermentation characteristics and microbiota in goats are examined.

The article with the title "Effect of dietary nitrate on enteric methane emissions, production performance and rumen fermentation of dairy cows grazing kikuyu-dominant pasture during summer", Van Wyngaard J.D.V. et al., Animal Feed Science and Technology, Elsevier, Amsterdam, NL, vol. 244, 13 August 2018, pages 76 - 87, describes a study on the effect of dietary nitrate on methane production from grazing dairy cows.

US 2,768,895 A discloses non-protein products which may be supplied to animal fodders at moderate or low cost and are capable of being converted to organismal protein by the metabolic processes of the host animal.

It is a goal of the present disclosure to provide NPN-sources for ruminants, such as for a ruminant feed supplement composition which overcomes the limitations of the known prior art, and which is particularly useful for improving the retention of N by rumen microorganisms and, at the same time, contributing to decrease enteric methane production.

## Summary

The invention is defined by the appended set of claims.

The present disclosure generally relates to a composition or mixture comprising urea, biuret, and one or more nitrate compounds. The terms "NPN composition", "NPN mixture", "composition comprising urea, biuret production out of urea" or "ruminant feed supplement composition" are generally used interchangeably herein, and refer to a composition or NPN composition according to the present disclosure, as further detailed herein.

The present inventors have surprisingly found that a specific composition for ruminants, comprising urea, biuret and one or more nitrate compounds, also referred herein as a "NPN composition" or "ruminant feed supplement composition" has many advantages, as further detailed below, when included in a ruminant feed composition or fed to a ruminant as a NPN source, particularly when compared to a ruminant fed with urea as a NPN source. Moreover, for the different embodiments and aspects envisaged herein, the composition according to the present disclosure significantly outperforms urea as a NPN source (when compared to a ruminant fed with a diet without NPN source).

According to a first aspect of the present disclosure, a composition, particularly a ruminant feed supplement composition is disclosed comprising urea, biuret and one or more nitrate compounds in an amount of between 5 and 60 wt.% of urea, between 2 and 60 wt.% of biuret, and between 20 and 40 wt. of one or more nitrate compounds, based on the total weight of the composition. Considering that supplemental protein contributes the largest proportion of the feeding costs in ruminant production, this supplement has the advantage to be a cost-effective source of N for controlling them. In particular, the composition comprising urea, biuret and one or more nitrate compounds according to the present disclosure improved nitrogen nutrition, such as by increasing the efficiency of microbial protein synthesis (EMPS) by rumen microbes in a ruminant animal, and it simultaneously increases the (in vitro) dry matter digestibility. Additionally, the added nitrate compound(s) simultaneously contributed to mitigate methane emissions by ruminants. This is at least partially due to feeding nitrates as a dietary NPN source decreases enteric methane production by outcompeting methanogenesis in the rumen when nitrate ($NO_3$) is reduced to ammonia ($NH_3$).

In a more specific embodiment of a ruminant feed supplement composition according to the present disclosure, the composition comprises between 20 and 40 wt.% of urea, between 20 and 40 wt.% of biuret and between 21 and 37 wt.% of one or more nitrate compounds is disclosed, based on the total weight of the composition.

In a still more specific embodiment of a ruminant feed supplement composition according to the present disclosure, the supplement comprises between 25 and 35 wt.% of urea, even more specifically between 29 and 33 wt.% of urea; between 25 and 35 wt.% of biuret, even more specifically between 29 and 33 wt.% of biuret, and between 23.5 and 31.5 wt.% of one or more nitrate compounds.

In a possible embodiment of a ruminant feed supplement composition according to the present disclosure, the nitrate compound is a water-soluble nitrate salt. More in particular the water-soluble nitrate salt is chosen out of calcium nitrate ($Ca(NO_3)_2$), magnesium nitrate ($Mg(NO_3)_2$), ammonium nitrate ($NH_4NO_3$), potassium nitrate ($KNO_3$) and/or sodium nitrate ($NaNO_3$). The nitrate present in these nitrate salts has a nutritional value for the ruminant as it is an NPN since they provide nitrogen for the microbial protein synthesis. Furthermore, the magnesium, calcium, potassium, sodium, etc. may have an additional nutritional value for the ruminants as these are macrominerals that can be utilized by the animal.

More in particular, the supplement comprises between 20 and 32 wt.% of calcium nitrate, still more in particular between 22 and 29 wt.% of calcium nitrate, and between 1 and 5 wt.% of ammonium nitrate, and still more in particular between 1.5 and 2.5 wt.% of ammonium nitrate.

In an optional embodiment of a ruminant feed supplement composition according to the present disclosure, the supplement comprises between 1 and 5 wt.% of triuret, more in particular between 1.5 and 3 wt.% of triuret.

In another optional embodiment of a ruminant feed supplement composition according to the present disclosure, the supplement comprises between 0.5 and 3.0 wt.% of ammelide, more in particular between 0.9 and 1.5 wt.% of ammelide.

In another optional embodiment of a ruminant feed according to the present disclosure, the supplement comprises between 3 and 30 wt.% of cyanuric acid, more in particular between 3 and 7 wt.% of cyanuric acid.

In a possible embodiment of a ruminant feed supplement composition according to the present disclosure, the supplement comprises between 0.1 and 4.5 wt.% of moisture, more in particular between 0.2 and 2.5 wt.% of moisture.

According to another aspect of the present disclosure, the use of a composition according to the present disclosure, particularly a ruminant feed supplement composition comprising urea, biuret and a nitrate compound, as a NPN-source to improve nitrogen nutrition and simultaneously to reduce enteric methane emissions of a ruminant animal is disclosed. It has been observed that the supplementation of a ruminant feed with a NPN composition comprising urea, biuret and one or more nitrate compounds according to the present disclosure, as a NPN source, reduces enteric methane emissions of ruminant animals without compromising or even improving the animal's performance, and simultaneously increases nitrogen nutrition, particularly when compared to a diet or ruminant feed supplemented with urea or a urea+biuret mixture as NPN source.

According to a related aspect of the present disclosure, the use of a composition according to the present disclosure, particularly a ruminant feed supplement composition comprising urea, biuret and a nitrate compound, as a NPN-source is disclosed for further, and in particular simultaneously:

- improving the efficiency of microbial protein synthesis (EMPS) by rumen microbes in a ruminant animal; and/or
- decreasing the acetate to propionate ratio produced by rumen microbes in a ruminant animal; and/or
- increasing the dry matter (DM) and and organic matter (OM) digestibility; and/or
- for improving the average daily gain (ADG) of a ruminant animal; and/or
- for improving the feed efficiency of a ruminant animal;

particularly when compared to a diet or ruminant feed supplemented with urea as NPN source.

In a particular use according to the present disclosure, the composition according to the present disclosure comprises between 5 and 60 wt% of urea, between 2 and 60 wt% of biuret and between 20 and 40 wt% of one or more nitrate compounds, based on the total weight of the composition, more in particular between 20 and 40 wt% of urea, between 20 and 40 wt% of biuret, and between 21 and 37 wt% of one or more nitrate compounds, based on the total weight of the composition; even more in particular between 25 and 35 wt.% of urea, between 25 and 35 wt.% of biuret and between 23.5 and 31.5 wt.% of one or more nitrate compounds, based on the total weight of the composition.

In a particular use according to the present disclosure, the nitrate compound is a water-soluble nitrate salt, more in particular chosen out of calcium nitrate, magnesium nitrate, ammonium nitrate, potassium nitrate and/or sodium nitrate. In a specific use according to the present disclosure, the composition comprises between 20 and 32 wt% of calcium nitrate and between 1 and 5 wt% of ammonium nitrate, based on the total weight of the composition.

In a further particular use according to the present disclosure, the composition according to the present disclosure further comprises between 1 and 5 wt.% of triuret, between 0.5 and 3 wt.% of ammelide, between 3 and 30 wt.% of cyanuric acid, and/or between 0.1 and 4.5 wt.% of moisture, with wt% based on the total weight of the composition.

In a possible use according to the present disclosure, a ruminant feed supplement composition as described above according to the present disclosure is used.

According to a further related aspect of the present disclosure, a method for simultaneously reducing the enteric methane emissions of a ruminant animal and improving nitrogen nutrition is disclosed, the method comprising administering to the ruminant animal a composition comprising urea, biuret and one or more nitrate compounds according to the present disclosure, particularly a ruminant feed supplement composition according to the present disclosure, as a NPN source, wherein the ruminant feed supplement composition comprises between 5 and 60 wt% of urea, between 2 and 60 wt% of biuret and between 20 and 40 wt% of one or more nitrate compounds, based on the total weight of the composition. In certain embodiments, the enteric methane emissions of the ruminant animal is reduced by at least 5%, such as by about 5 to 10%, when the composition is administered to a ruminant as NPN source compared to a diet comprising urea as NPN source.

According to a particular embodiments, said method is further and in particular simultaneously:

- a method for improving the efficiency of microbial protein synthesis (EMPS) by rumen microbes in a ruminant animal; and/or
- a method for decreasing the acetate to propionate ratio produced by rumen microbes in a ruminant animal; and/or
- a method for increasing the dry matter (DM) and organic matter (OM) digestibility; and/or
- a method for improving the average daily gain (ADG) of a ruminant animal; and/or
- a method for improving the feed efficiency of a ruminant animal.

According to particular embodiments of the method according to the present disclosure, the composition according to the present disclosure comprises between 20 and 40 wt% of urea, between 20 and 40 wt% of biuret, and between 21 and 37 wt% of one or more nitrate compounds, based on the total weight of the composition; even more in particular between 25 and 35 wt.% of urea, between 25 and 35 wt.% of biuret and between 23.5 and 31.5 wt.% of one or more nitrate compounds, based on the total weight of the composition.

In a particular method according to the present disclosure, the nitrate compound is a water-soluble nitrate salt, more in particular chosen out of calcium nitrate, magnesium nitrate, ammonium nitrate, potassium nitrate and/or sodium nitrate. In a specific method according to the present disclosure, the composition comprises between 20 and 32 wt% of calcium nitrate and between 1 and 5 wt% of ammonium nitrate, based on the total weight of the composition.

In a further particular method according to the present disclosure, the composition further comprises between 1 and 5 wt.% of triuret, between 0.5 and 3 wt.% of ammelide, between 3 and 30 wt.% of cyanuric acid, and/or between 0.1 and 4.5 wt.% of moisture,
with wt% based on the total weight of the composition.

In certain embodiments of the methods according to the present disclosure, the method comprises the steps of preparing or providing a ruminant feed composition or a ruminant feed supplement composition comprising the composition comprising urea, biuret and one or more nitrate compounds according to the present disclosure as a NPN source, and administering the ruminant feed supplement or the ruminant feed composition to the ruminant animal. In particular, the composition comprising urea, biuret and one or more nitrate compounds according to the present disclosure is present in the ruminant feed composition in a concentration between 0.5 and 4 wt.%, more in particular between 0.5 and 3.5 wt.% or between 0.5 and 3 wt.%, even more in particular between 1 and 2.5 wt.%, with wt% based on the total dry matter basis of the ruminant feed composition.

## Description of the figures

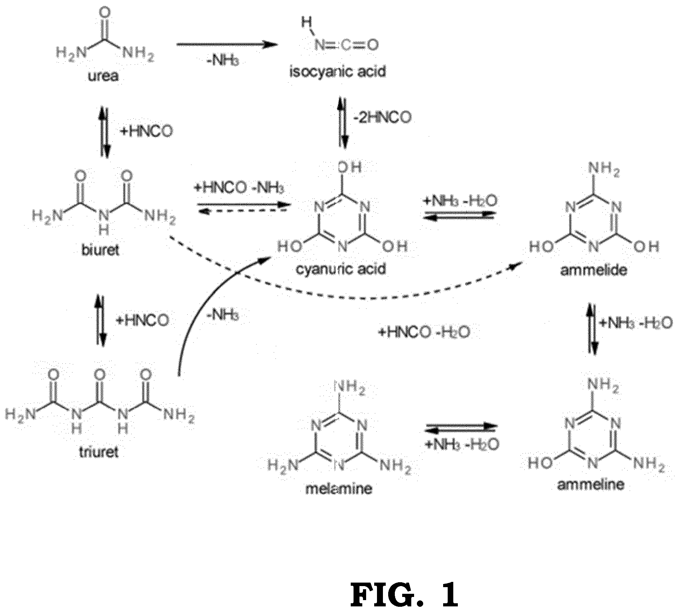

FIG. 1 shows an overview of the reactions involved in the production of biuret from urea, including the formation of by-products.

## Detailed description

The present inventors have surprisingly found that specific NPN compositions for ruminant animals, comprising urea, biuret, and one or more nitrate compounds, and further in particular also comprising one or more N-containing by-products of a urea condensation process or a biuret production process, have many advantages when incorporated in the diet of ruminant animals and thus are fed to the ruminant animals as a NPN source, particularly when compared to a ruminant fed with urea as a NPN source. Moreover, for the different embodiments and aspects envisaged herein, the composition according to the present disclosure comprising urea, biuret and one or more nitrate compounds, also referred herein as "NPN composition" or "ruminant feed supplement composition", particularly comprises between 5 and 60 wt% of urea, between 2 and 60 wt% of biuret and between 20 and 40 wt% of one or more nitrate compounds, based on the total weight of the composition, significantly outperforms urea as a NPN source (when compared to a ruminant fed with a diet without NPN source).

The advantages of a composition comprising urea, biuret and one or more nitrate compounds according to the present disclosure are in particular:

- the composition acts as a dietary NPN-source for ruminants thereby improving the nitrogen nutrition, and at the same time reduces enteric methane emissions in ruminants. The reduction of the methane production or emissions by ruminant animals can be assessed *in vivo*, by determining the methane emissions of a ruminant fed with a particular diet, for instance using using the $SF_6$ tracer technique as e.g. described in example 2, wherein the methane emissions may be expressed as g methane emitted per kg dry matter intake or per kg organic matter intake. Alternatively, the methane emissions may be assessed via *in vitro* culture incubations of ruminal fluid, as e.g. described in example 1, wherein the methane emissions may be expressed as mM, ml or mg methane produced per g of organic matter incubated or digested.

- the average daily gain (ADG) of ruminant animals was improved. This is an important parameter for farmers, as it represents how quickly the animal is gaining weight. The faster an animal gains, the quicker it is ready for market, which can decrease costs;
- the feed efficiency of ruminant animals was improved;
- the efficiency of microbial protein synthesis (EMPS) by rumen microbes in a ruminant animal was increased. As understood by the skilled person, the EMPS is a measure for how much microbial protein is produced due to the microbial activity for every unit of fermentable substrate which is fed to the ruminant. It may be determined by an *in vitro* assay comprising continuous flow or batch fermentations with rumen fluid, particularly wherein a suitable fermenter or incubator is inoculated with ruminal fluid and fed with a ruminant feed composition to simulate rumen fermentation; as for instance explained in example 1;
- the dry matter (DM) and organic matter (OM) digestibility was increased. The DM and OM digestibility may be assessed by the difference in dry matter and organic matter (i.e. dry matter minus the ash content) of the feed vs the sample after (in vitro) fermentation, as known by the skilled person;
- the dry matter intake (DMI) of a ruminant animal was increased;
- the acetate to propionate ratio produced by rumen microbes in a ruminant animal was decreased. The acetate and propionate content can be determined by gas chromatography determination of the volatile fatty acids (VFA) in a sample, as known by the skilled person. In certain embodiments, the sample is obtained via an *in vitro* fermentation with ruminant fluid, to simulate rumen fermentation, particularly wherein a suitable fermenter or incubator is inoculated with ruminal fluid and fed with a ruminant feed composition to simulate rumen fermentation

In this context, and without wishing to be bound by theory, it is believed that there has been a selective pressure on the microbes in the rumen that produce acetate and propionate in such a way that more propionate and less acetate, in view of ruminant feed supplement compositions without the mixture according to the present disclosure, are produced. Propionate is the most important glucogenic precursor in ruminants. This means that lowering the acetate to propionate ratio enhances the supply of energy for metabolic use, through which more energy is available to be used by the ruminant enhancing its production of proteins. In other words, the rumen fermentation has been made more efficient in an energetic way, supporting the production of meat, milk, etc.

Biuret is typically formed through a non-catalytic thermal decomposition of urea. This process is generally referred to herein as "biuret production", "biuret production out of urea" or "urea condensation process". The main reaction and the side reactions in biuret production out of urea are known to the skilled person and they are represented in FIG. 1. When urea is heated to temperatures above its melting point, ammonia is slowly evolved. If the temperature range is limited slightly above the normal melting point of urea, i.e. 132 °C, biuret is formed. When heating the urea melt further, several other different "N-containing compounds" or "N-containing by-products" are formed in very small amounts - hence use of the term "minor". These N-containing compounds resulting out of the thermal decomposition of urea encompass amongst others cyanuric acid, triuret, ammelide, etc. These compounds are referred to herein as "N-containing by-products of biuret production" or as "minor N-containing compounds created during the urea condensation process". In particular embodiments, said N-containing by-products of biuret production comprise triuret, ammelide and cyanuric acid. These by-products are poorly hydrolysed in the rumen. Cyanuric acid and triuret are, just like biuret, non-toxic for the ruminants and give as good nitrogen retention figures as biuret does. Ammelide is an unavoidable by-product in the production of the necessary amount of biuret. The amount of ammelide however needs to be limited since it can be toxic in higher doses.

As used herein, the composition or mixture according to the present disclosure, in particular the NPN composition or mixture according to the present disclosure, generally comprises urea, biuret and one or more nitrate compounds.

The present disclosure firstly relates to a composition, particularly a NPN composition or a ruminant feed supplement composition which comprises urea, biuret and one or more nitrate compounds. A ruminant feed supplement composition is meant to be included as a part of the ruminant diet. Not being bound by any theory, the combination of urea, biuret and one or more nitrate compounds provides an intrinsic slow release effect in the gastrointestinal tract of the animal.

More in particular, the NPN composition, particularly the ruminant feed supplement composition, i.e. the composition comprising urea, biuret and one or more nitrate compounds according to the present disclosure, comprises:

- between 5 and 60 wt.%, more in particular between 20 and 40 wt.% of urea, even more in particular between 25 and 35 wt.%, and yet even more in particular between 29 and 33 wt.% of urea;
- between 2 and 60 wt.%, more in particular between 20 and 40 wt.% of biuret, even more in particular between 25 and 35 wt.%, and yet even more in particular between 29 and 33 wt.% of biuret;
- between 20 and 40 wt.%, more in particular between 21 and 37 wt.% and even more in particular between 23.5 and 31.5 wt.% of one or more nitrate compounds.

Accordingly, the ruminant feed supplement composition or NPN composition according to the present disclosure comprises between 5 and 60 wt% of urea, between 2 and 60 wt% of biuret and between 20 and 40 wt% of one or more nitrate compounds, based on the total weight of the composition; more in particular comprises between 20 and 40 wt% of urea, between 20 and 40 wt% of biuret, and between 21 and 37 wt% of one or more nitrate compounds, based on the total weight of the composition; even more in particular comprises between 25 and 35 wt.% of urea, between 25 and 35 wt.% of biuret and between 23.5 and 31.5 wt.% of one or more nitrate compounds, based on the total weight of the composition. The one or more nitrate compounds are more in particular water-soluble nitrate salts. Examples thereof are calcium nitrate, magnesium nitrate, ammonium nitrate, potassium nitrate, sodium nitrate or a combination of two or more of these.

The NPN composition or ruminant feed supplement composition more in particular comprises between 20 and 32 wt.% of calcium nitrate and between 1 and 5 wt.% of ammonium nitrate, more in particular between 22 and 29 wt.% of calcium nitrate and between 1.5 and 3.0 wt.% or between 1.5 and 2.5 wt.% of ammonium nitrate.

It is remarked that the amount of the one or more nitrate compounds and the type thereof must be such that these are not toxic to the ruminant.

More in particular, the NPN composition or ruminant feed supplement composition according to the present disclosure further comprises low percentage of one or more N-containing by-products of a urea condensation process or a biuret production process, such as cyanuric acid, triuret and ammelide, which are poorly hydrolysed in the rumen. Cyanuric acid and triuret are, just like biuret, non-toxic for the ruminants and give as good nitrogen retention figures as biuret does. Ammelide is an unavoidable by-product in the production of the necessary amount of biuret. The amount of ammelide however needs to be limited since it can be toxic in higher doses. More in particular, the NPN composition or ruminant feed supplement composition according to the present disclosure further comprises

- between 1 and 5 wt.%, more in particular between 1.5 and 3 wt.% of triuret;
- between 0.5 and 3 wt.%, more in particular between 0.9 and 1.5 wt.% of ammelide; and
- between 3 and 7 wt.%, more in particular between 3 and 7 wt.% of cyanuric acid.

The ruminant feed supplement composition or the composition comprising urea, biuret and one or more nitrate compounds according to the present disclosure further comprises more in particular between 0.1 and 4.5 wt.%, and even more in particular between 0.2 and 2.5 wt.% of moisture ($H_2O$).

More in particular, the ruminant feed supplement composition or the composition comprising urea, biuret and one or more nitrate compounds according to the present disclosure comprises:

- between 5 and 60 wt.%, more in particular between 20 and 40 wt.% of urea, even more in particular between 25 and 35 wt.%, and yet even more in particular between 29 and 33 wt.% of urea;
- between 2 and 60 wt.%, more in particular between 20 and 40 wt.% of biuret, even more in particular between 25 and 35 wt.%, and yet even more in particular between 29 and 33 wt.% of biuret;
- between 20 and 40 wt.%, more in particular between 21 and 37 wt.% and even more in particular between 23.5 and 31.5 wt.% of one or more nitrate compounds, such as between 20 and 32 wt.% of calcium nitrate and between 1 and 5 wt.% of ammonium nitrate, more in particular between 22 and 29 wt.% of calcium nitrate and between 1.5 and 2.5 wt.% of ammonium nitrate;
- between 1 and 5 wt.%, more in particular between 1.5 and 3 wt.% of triuret;
- between 0.5 and 3 wt.%, more in particular between 0.9 and 1.5 wt.% of ammelide;
- between 3 and 7 wt.%, more in particular between 3 and 7 wt.% of cyanuric acid; and
- between 0.1 and 4.5 wt.%, and even more in particular between 0.2 and 2.5 wt.% of moisture ($H_2O$).

Optionally, for all embodiments of the ruminant feed supplement composition or the composition comprising urea, biuret and one or more nitrate compounds according to the present disclosure, one or more anti-caking agents as generally known in the art can be added to the ruminant feed supplement composition, either incorporated in the feed supplement, either as a coating, to improve handling properties and to improve the quality of the ruminant feed supplement composition.

Furthermore, it is also possible to add any pre-biotics, pro-biotics, post-biotics, anti-biotics, bio stimulants known in the art for fostering / supporting the nitrogen assimilation and rumen fermentation.

The present disclosure further relates to the use of the NPN composition according to the present disclosure or ruminant feed supplement composition comprising urea, biuret and one or more nitrate compounds according to the present disclosure as a dietary NPN-source for ruminants to improve nitrogen nutrition and at the same time to reduce the enteric methane emissions or production in a ruminant animal, and, also, in particular embodiments simultaneously, to:

- increase the efficiency of microbial protein synthesis (EMPS) by rumen microbes in a ruminant animal;
- decrease the acetate to propionate ratio produced by rumen microbes in a ruminant animal;
- increase the dry matter (DM) and organic matter (OM) digestibility;
- increase the average daily gain (ADG) of a ruminant animal;
- increasing the dry matter intake (DMI) of a ruminant animal;
- increase the feed efficiency of a ruminant animal.

According to a related aspect of the present disclosure, a NPN composition comprising urea, biuret and one or more nitrate compounds according to the present disclosure for use as an NPN-source in a ruminant feed supplement composition to decrease the enteric methane production or emissions of a ruminant animal or, stated differently, to decrease the enteric methane production by rumen microbes in a ruminant, and simultaneously to improve nitrogen nutrition is disclosed.

As microbial proteins are the major source of protein in the diet of a ruminant, as explained in example 1, section "microbial protein synthesis", the improved nitrogen nutrition for the composition according to the present disclosure is related to an improved microbial growth and fermentation, which in its turn can be expressed by the efficiency of microbial synthesis (EMPS) and the DM and/or OM digestibility. In this context, it has advantageously been found that a composition comprising urea, biuret and one or more nitrate compounds according to the present application, when used as a NPN source in a ruminant diet supported the *in vitro* microbial fermentation to a greater extent than urea or a urea/biuret mixture as NPN source, thus indicating that the NPN composition according to the present application also supports the microbial fermentation in the rumen to a greater extent than urea. Accordingly, in certain embodiments, the present disclosure relates to the use of the NPN composition or ruminant feed supplement composition c according to the present disclosure as a dietary NPN-source for ruminants to simultaneously reduce the enteric methane production in a ruminant animal, and to increase the efficiency of microbial protein synthesis (EMPS) by rumen microbes in a ruminant animal.

Also, in certain embodiments, the present disclosure relates to the use of the composition or ruminant feed supplement composition comprising urea, biuret and one or more nitrate compounds according to the present disclosure as a dietary NPN-source for ruminants to simultaneously reduce the enteric methane production in a ruminant animal and to increase the dry matter (DM) and organic matter (OM) digestibility.

As the ADG is an important parameter to the farmer, in certain embodiments, the present disclosure relates to the use of the composition or ruminant feed supplement composition comprising urea, biuret and one or more nitrate compounds according to the present disclosure as a dietary NPN-source for ruminants to simultaneously reduce the enteric methane production in a ruminant animal and to increase the average daily gain (ADG) of a ruminant animal.

According to a related aspect of the present disclosure, a composition comprising urea, biuret and one or more nitrate compounds according to the present disclosure for use as an NPN-source in a ruminant feed supplement composition to decrease the ratio of acetate to propionate produced by rumen microbes in a ruminant is disclosed.

According to a further related aspect of the present disclosure, a composition comprising urea, biuret and one or more nitrate compounds according to the present disclosure for use as an NPN-source in a ruminant feed supplement composition to improve the EMPS in a ruminant is disclosed.

According to a further related aspect of the present disclosure, a composition comprising urea, biuret and one or more nitrate compounds according to the present disclosure for use as an NPN-source in a ruminant feed supplement composition to increase the DM and OM digestibility in a ruminant.

According to a related aspect of the present disclosure, a composition comprising urea, biuret and one or more nitrate compounds according to the present disclosure for use as an NPN-source in a ruminant feed supplement composition to increase the average daily gain (ADG) of a ruminant animal and/or to increase the feed efficiency of a ruminant animal is disclosed.

A related aspect of the present disclosure provides a method for reducing the enteric methane emissions of a ruminant animal and for simultaneously improving nitrogen nutrition, particularly when compared to a diet or ruminant feed supplemented with urea as NPN source, and optionally further for improving the efficiency of microbial protein synthesis (EMPS) by rumen microbes in a ruminant animal; for decreasing the acetate to propionate ratio produced by rumen microbes in a ruminant animal; for increasing the dry matter (DM) and organic matter (OM) digestibility; for improving the average daily gain (ADG) of a ruminant animal; for improving the feed efficiency of a ruminant animal; said method comprising the steps of

- preparing a ruminant feed composition comprising a NPN composition according to the present disclosure, as a NPN source, particularly a NPN composition or ruminant feed supplement composition comprising urea, biuret and one or more nitrate compounds according to the present disclosure; and

- feeding a ruminant with the ruminant feed composition.

In certain embodiments, the enteric methane emissions of a ruminant animal is reduced by at least 5%, such as by about 5% to 10%, for instance when expressed in g/kg DMI (dry matter intake) or g/kg OMI (organic matter intake), when the composition comprising urea, biuret and one or more nitrate compounds according to the present disclosure is administered to a ruminant as NPN source, compared to diet comprising urea or a urea/biuret mixture as NPN source. In certain embodiments, the enteric methane production of a ruminant animal, when estimated via an *in vitro* culture incubation of ruminal fluid, as e.g. described in example 1, is reduced by at least 5%, such as by between about 5% and 10% when expressed in mM/g OMi (organic matter incubated) or in mM/g OMd (organic matter digested), when the composition comprising urea, biuret and one or more nitrate compounds according to the present disclosure is administered as NPN source compared to diet comprising urea as NPN source.

In certain embodiments, the average daily gain (ADG) of a ruminant animal is increased by at least 5%, more in particular by at least 10%, or even more in particular by at least 15%, such as upto 20% or more, when the ruminant feed supplement composition is administered to a ruminant as NPN source compared to a diet comprising urea as NPN source.

In certain embodiments, EMPS in a ruminant is increased by at least 5%, in particular by at least 10% or by at least 15%, such as upto 30% and more, when the composition comprising urea, biuret and at least one nitrate compound according to the present disclosure is administered to a ruminant as NPN source compared to a diet without NPN source. In particular, the EMPS may be determined on a sample obtained from an *in vitro* fermentation with ruminant fluid, to simulate rumen fermentation, particularly wherein a suitable fermenter or incubator, such as a continuous flow or batch fermenter or incubator, is inoculated with ruminal fluid and fed with a ruminant feed composition to simulate rumen fermentation.

In certain embodiments, the (in vitro) OM and DM digestibility in a ruminant is increased by at least 5%, such as by between about 5 and 10%, when the composition comprising urea, biuret and at least one compound according to the present disclosure is administered to a ruminant as NPN source compared to a diet comprising urea as NPN source. In particular, the OM and DM digestibility may be determined on a sample obtained from an *in vitro* fermentation with ruminant fluid, to simulate rumen fermentation, particularly wherein a suitable fermenter or incubator, such as a continuous flow or batch fermenter or incubator, is inoculated with ruminal fluid and fed with a ruminant feed composition to simulate rumen fermentation.

In certain embodiments, the method for decreasing the enteric methane production of a ruminant animal, for decreasing the acetate to propionate ratio produced by rumen microbes in a ruminant, for improving the EMPS in a ruminant, for increasing the DM or OM digestibility in a ruminant, and/or for increasing the dry matter intake (DMI) of a ruminant further comprises the step of replacing a portion of vegetable proteins in a feed composition with the ruminant feed supplement composition comprising urea, biuret and one or more nitrate compounds according to the present disclosure.

It is further understood that the different methods according to the present disclosure are non-therapeutic methods.

As considered herein a ruminant or a ruminant animal is a bovine, ovine or caprine animal, particularly a domesticated animal, with a functional rumen. In particular embodiments, the ruminant is a sheep or a cattle, more in particular the ruminant is a beef cattle or dairy cattle.

The present disclosure further provides a feed, feed material, premix or feed additive for feeding a ruminant comprising the composition comprising urea, biuret and at least one nitrate compound according to the present disclosure. The composition according to the present disclosure may be combined with a ruminant feed or feed material, or with a premix for feeding a ruminant. In context of the present disclosure the term premix or nutrient premix is used as known to the person skilled in the art and denotes a mixture comprising one or more ingredients such as vitamins, trace minerals, medicaments, feed supplements and diluents. A ruminant feed or ruminant feed material generally refers to nutritional compositions suitable for feeding to ruminants, as known to the skilled person, and includes compound feeds (i.e. generally an industrially produced feed mixture of at least two feed materials), complete feeds (i.e. a compound feed containing all main nutrients needed for a daily ration, including also all the roughage needed by the ruminant), or a concentrate feed (i.e. a compound feed which has a high content of certain substances but which is sufficient for a daily ration only if used in combination with other feed).

In particular embodiments, a ruminant feed composition comprising one or more nutritional ingredients and a NPN composition according to the present disclosure is disclosed, wherein the NPN composition comprises between 5 and 60 wt% of urea, between 2 and 60 wt% of biuret and between 20 and 40 wt% of one or more nitrate compounds, based on the total weight of the composition, more in particular between 20 and 40 wt% of urea, between 20 and 40 wt% of biuret, and between 21 and 37 wt% of one or more nitrate compounds, based on the total weight of the composition; even more in particular between 25 and 35 wt.% of urea, between 25 and 35 wt.% of biuret and between 23.5 and 31.5 wt.% of one or more nitrate compounds, based on the total weight of the composition. In certain embodiments, the one or more nutritional ingredients form a nutritionally balanced ration or a basal ration for a ruminant, as known by the skilled person.

In certain embodiments, a ruminant feed composition or feed material comprising between 0.5 and 4 wt.%, more in particular between 0.5 and 3.5 wt.% or between 0.5 and 3 wt.%, even more in particular between 1 and 2.5 wt.% of the NPN composition according to the present disclosure comprising urea, biuret and one or more nitrate compounds, is disclosed, with wt.% based on the total dry matter of the feed composition or feed material,

The present disclosure further relates to a method for feeding or rearing a ruminant comprising administering the composition comprising urea, biuret and one or more nitrate compounds according to the present to the ruminant, particularly during the feeding regime, and/or feeding the ruminant with the ruminant feed composition according to the present disclosure.

The disclosure as defined here above will now be illustrated and explained in more detail in the following experimental part, which is not intended to limit the scope of this disclosure anyway.

<u>Examples</u>

***EXAMPLE 1: Effects of different proportions of NPN-mixture comprising biuret, urea and nitrates on in vitro ruminal fermentation and methane production***

In this example, an *in vitro* assay was performed using batch culture incubations, aiming to simulate rumen fermentation.

***Animal adaptation***

Two ruminally cannulated Angus crossbred steers (808.8 $\pm$ 36.3 kg body weight) were used as ruminal fluid donors for the in vitro batch culture incubations. The steers were fed a corn silage and gin trash diet (70% and 30% on a dry matter basis, respectively) ad libitum at least 35 days before collecting ruminal fluid to perform the in vitro incubations. Each steer received a NPN-mixture equivalent to 100 g of urea per day comprised of an equal amount of N from the different NPN sources to adapt the rumen microbial community. Thus, each steer was fed daily 33 g of urea (Rumisan®, 46 wt.% N, Yara International ASA), 37 g of a urea-biuret mixture comprising 41 wt.% N and 97 g of calcium nitrate (CN) (Bolifor® CNF, 15,5 wt.% N, Yara International ASA). These amounts were mixed in with the feed diet every two days. The nitrate source (Bolifor® CNF) was gradually introduced to the feed diet as follows: 29 g/ animal / day during the first week, 58 g / animal / day during the second week, and 97 g / animal / day from the third week until the end of the experiment.

***Feed preparation and chemical analysis***

The feed was prepared by drying corn silage and gin trash for 45 hours at 55°C and ground to pass a 2 mm screen in a Wiley mill.

***Experimental design and treatments***

The in vitro batch culture incubations were conducted as a randomized complete block design using 70 wt.% corn silage and 30 wt.% gin trash on a dry matter basis conserving the diet characteristics used during the animal adaptation period. The treatments were designed to be isonitrogenous (= having the same amount of dietary nitrogen) and equivalent to 1 wt.% of urea inclusion. The treatments were consequently as follows:

- control without NPN-supplementation (1) - in the examples generally referred to as "Control";
- 100 wt.% urea (Rumisan®) (2) - in the examples generally referred to as "Urea";
- four different proportions of a mixture of urea and biuret and nitrates (3) - in the examples generally referred to as "UBN". The 4 different proportions urea+biuret:nitrate are 100:0 (UBN 0: comparative example), 75:25 (UBN 1), 50:50 (UBN 2: comparative example), 25:75 (UBN 3: comparative example).

***Rumen fluid collection and in vitro incubations***

The in vitro incubations were conducted on three separate days (replicates). A representative sample of digesta was collected from different places in the rumen of ruminally cannulated steers and strained through 4 layers of cheesecloth, placed in pre-warmed thermos containers, and transported to the laboratory within 30 minutes after the collection. In the laboratory, the ruminal fluid from the two steers was maintained under constant $CO_2$-flux and was combined in equal proportions. This combined ruminal fluid was mixed with McDougall's buffer (McDougall, 1948) with and without 0.077 g/liter of 10 wt.% ammonium sulfate enriched with $^{15}$N in relation 1:4, and it was used as an inoculum. Two bottles of 500 ml fitted with a side arm and a rubber septum were incubated per treatment. The bottles contained 5.6 g of substrate dry matter and 400 ml of $^{15}N$ enriched inoculum, and were incubated for 24 hours at 39°C with an agitation of 60 rpm to monitor gas production kinetics using an Ankom Gas Monitoring System from Ankom Technologies, Fairport, New York. Also, one 500-ml bottle without $^{15}N$ enriched inoculum was incubated to determine the basal $^{15}N$ enrichment. At 12 hours of incubation, two samples of 10 ml of rumen fluid were collected from each bottle through the septa port using a 20-ml syringe. These samples were acidified by adding 10 μl of a 20 vol/vol% of $H_2SO_4$-solution to each 10-ml sample, and frozen at minus 20°C until further analysis. At the end of the 24 hours incubation, the final pH was recorded, the bottles were placed on ice to prevent further microbial fermentation and two 10-ml samples were collected and acidified by adding 20 μl of a 20 vol/vol% of $H_2SO_4$-solution and were frozen at minus 20°C until further analysis. The remaining fermentation content was thereafter agitated at 60 rpm for 30 seconds and divided into two subsamples. From the first subsample, composed of whole contents, a volume of 180 ml was frozen at minus 20°C until further analysis and from the second subsample 180 ml were used to recover the microbial pellet. Furthermore, two tubes of 100 ml per treatment containing 0.7 g of the substrate on a dry matter basis and 50 ml of inoculum without $^{15}N$ enrichment and two blanks were incubated for 24 hours at 39 °C with agitation at a speed of 60 rpm. After the 24 hours incubation with ruminal fluid, 6 ml of HCl and 2 ml of a 5% pepsin solution were added. The tubes were incubated for another 48 hours at 39°C. After the incubation period, the tubes were maintained on ice and later stored for further analysis.

### Concentration of volatile fatty acids (VFA)

VFA produced by microbial fermentation in the rumen are the main source of energy absorbed by the digestive tract of ruminants. The amount and the profile of VFA in the rumen has consequences for the efficiency of energy utilization, production of methane, risks of ruminal acidosis (characterized by a decrease in pH caused by an accumulation of organic acids in the rumen and a shift in the delicate balance of rumen microorganisms). A water-based solution using ethyl acetate extraction was used to determine VFA concentrations at 12 and 24 hours. Samples were centrifuged at 10,000 × g for 15 min at 4°C, 2 ml of the supernatant were mixed with 0.4 ml (5:1 ratio) of a metaphosphoric-crotonic acid solution, and samples were frozen overnight. Samples were then thawed and centrifuged again at 10,000 × g for 15 min at 4°C. The supernatant was transferred into 12 × 75 mm borosilicate disposable culture tubes (Fisherbrand; Thermo Fisher Scientific Inc., Waltham, MA) and mixed with ethyl acetate to form a 2:1 ethyl acetate : supernatant mixture. The samples were analyzed with a gas chromatograph (Agilent 7820A GC, Agilent Technologies) using a flame ionization detector and a capillary column (CP-WAX 58 FFAP 25 m × 0.53 mm, Varian CP7767; Varian Inc.).

### Microbial protein synthesis

The rumen microbial ecosystem is made up mainly by species of bacteria, fungi, and protozoa. The microbes ferment ingested feeds by the ruminant, and, by fermentation, produce end products that are utilized by the ruminant as well as by the microbes themselves for their own proliferation and growth. The species diversity and their relative proportions within the microbial community depend primarily on the diet consumed by the animal. It is thus important that a healthy mixture of different feedstuffs is fed to the animal to keep the rumen and its microflora functioning efficiently. The speed of digestion of the feed depends on the quality and the supplement of the feed and is affected by the number and type of microbes, the pH in the rumen, the nutrients limiting the growth of the microbes and the removal of microbes from the rumen. Energy and protein are the major nutrients that limit the microbial growth, and therefore, the rumen fermentation. The microbial population needs energy and protein for their growth and multiplication. Protein, when digested, is broken down into peptides, which are short chains of amino acids. Further digestion of peptides results in individual amino acids and eventually ammonia. Rumen microbes are the major source of protein for the ruminant animal. If there is enough energy available, the rumen microbes break down the rumen degradable protein to amino acids and then to ammonia which is a major N-source for microbial growth. The microbes also convert NPN to ammonia, which contributes to the ammonia pool available for microbial growth. Microbial protein is continuously flushed from the rumen into the intestine where undergoes enzymatic digestion. The amino acids produced from the digested microbial protein are absorbed through the small intestine. The amount of microbial protein flowing into the intestines therefore largely depends on the availability of fermentable energy and N in the diet consumed by ruminants.

The total nitrogen (N) and the isotopic supplement were measured in the whole content and the bacterial pellet by an isotopic ratio mass spectrometry using a Vario Micro cube from Elementar Analyzer System GmbH. The bacteria pellet subsample was slowly centrifuged at 1,000 x g for 10 minutes at 4°C. The resulting supernatant was fast centrifuged at 20,000 x g for 20 minutes at 4°C to obtain the pellet of bacteria. This bacteria pellet was then washed with a 0.9% saline solution and centrifuged at 20,000 x g for 20 minutes at 4°C, discarding the supernatant. The saline wash procedure was repeated twice. Finally, the bacterial pellet was freeze-dried. Separately, the 180-ml whole content subsample was completely freeze-dried. The dried samples of the whole content and of the bacterial pellet were manually grinded and weighted into an 8 x 5-mm pressed standard-weight tin capsule using a Mettler-Toledo Excellence Plus XP Micro Balance from Mettler-Toledo GmbH, Laboratory and Weighing Technologies. Then, 35 $\mu$l of a 10 g/l solution of $K_2CO_3$ were added to each sample and dried overnight in a forced-air oven at 60°C for complete $NH_3$-N evaporation. The percentage of atom $^{15}$N in the dried samples was analyzed in an isotope ratio mass spectrometer (IsoPrime 100 from IsoPrime) and expressed as the fractional abundance of isotopic fractions ($^{15}$N / $^{14}$N) multiplied by 10.

### Concentration of ammonia-N

Ruminal ammonia production largely reflects the amount of degradable protein in the rumen. Ruminal ammonia nitrogen (ammonia-N) is a critical nutrient for supporting microbial growth and fermentation. Ensuring the availability of ammonia-N in the rumen both on a quantitative and timely manner is fundamental for achieving maximum rate of fermentation and feed digestibility.

The concentration of ammonia-N at 12 and 24 hours in the incubation fluid was measured after centrifuging at 10,000 x g for 15 minutes at 4°C using an Avanti J-E centrifuge from Beckman Coulter® Incorporated. One ml of phenol reagent was pipetted into 12 × 75 mm borosilicate disposable culture tubes (Fisherbrand; Thermo Fisher Scientific Inc., Waltham, MA). A 20-$\mu$l aliquot of the supernatant from the centrifuged sample was transferred to the phenol-containing culture tubes. After vortexing, 0.8 ml of hypochlorite solution was added to the mixture and was vortexed again. The culture tubes were covered with glass marbles and placed in a water bath at 95°C for 5 minutes. The absorbance was read in 96-well, flat-bottom plates at a wavelength of 665 nm using a DU-500 plate reader from Beckman Coulter® Inc. The fermentation residue reserved into the tubes of 100 ml were filtered, dried at 105°C in a forced air oven for 24 hours and ashed at 550°C for 6 hours to determine the undigested organic matter.

### Nutrient disappearance

Digestibility represents the proportion of ingested nutrients consumed that are absorbed by the animal. Dry matter is defined as the loss of weight of samples when dried in an oven above 100°C during 12 - 24 hours, whereas organic matter defines the weight loss of the dry matter when combusted (the dry matter minus the ash content). Dry matter consists of all nutrients, whereas organic matter consists of all nutrients with the exception of ash. Organic matter digestibility is defined as the proportion of organic matter in the feed that is digested in the rumen or total ruminant digestive tract. Ruminal organic matter digestibility can be used to estimate energy available for fueling microbial growth and the resulting synthesis of microbial protein in the rumen.

The in vitro dry organic matter digestibility (IVDMD) and the in vitro organic matter digestibility (IVOMD) was calculated with the following formulae:

$$IVDMD\ (\%) = \frac{incubated\ dry\ matter - residual\ dry\ matter}{incubated\ dry\ matter}\ x\ 100$$

$$IVOMD\ (\%) = \frac{incubated\ organic\ matter - residual\ organic\ matter}{incubated\ organic\ matter}\ x\ 100$$

### Results

In Table 1 below, the concentration of the total VFA and the concentration of ammonia-N in: a control diet without NPN supplementation ("control"), a corn silage-based diet either with urea supplementation ("urea") or with supplementation of different urea-biuret-nitrate mixtures ("UBN"), i.e. UBN 0, UBN 1, UBN 2, UBN 3, with a ratio of urea + biuret versus nitrates of 100 : 0; 75 : 25; 50 : 50; and 25 : 75 respectively, after 12 hours of incubation is shown. In Table 2 below, the same is shown after 24 hours of incubation. In Table 2, also the pH of the fluid, the in vitro dry matter degradability (IVDMD) and the in vitro organic matter degradability (IVOMD) after 24 hours of incubation are shown. The pH of the rumen has profound effects on the growth of the ruminal microbes and the digestion that takes place in this forestomach. Rumen pH can affect the extent of rumen fermentation by altering microbial populations and tissue absorptive capacity. Low ruminal pH for prolonged periods each day for instance can affect feed intake, microbial metabolism and feed digestion, and has also been related to inflammation, diarrhea and milk fat depression.

*Table 1: Effect of NPN supplementation on VFA and ammonia-N at 12 hours of incubation in a corn silage-based diet*

| Variable | Control | Urea | UBN mixtures | | | |
|---|---|---|---|---|---|---|
| | | | UBN 0 100 : 0 | UBN 1 75 : 25 | UBN 2 50 : 50 | UBN 3 25 : 75 |
| mM | | | | | | |
| Total VFA | 30.95 | 31.77 | 32.55 | 32.92 | 32.13 | 33.53 |
| Ammonia-N | 0.35 | 2.74 | 2.61 | 2.72 | 2.31 | 2.44 |

*Table 2: Effect of NPN supplementation on VFA and ammonia-N, pH and in vitro digestibility or organic and dry matter at 24 hours of incubation in a corn silage-based diet*

| Variable | Control | Urea | UBN mixtures | | | |
|---|---|---|---|---|---|---|
| | | | UBN 0 100 : 0 | UBN 1 75 : 25 | UBN 2 50 : 50 | UBN 3 25 : 75 |
| mM | | | | | | |
| Total VFA | 41.39 | 40.96 | 42.85 | 43.58 | 42.86 | 43.50 |
| Ammonia-N | 2.48 | 6.55 | 7.34 | 7.62 | 6.34 | 6.55 |
| pH | 6.34 | 6.38 | 6.37 | 6.36 | 6.35 | 6.39 |
| IVDMD, % | 45.02 | 45.08 | 45.78 | 48.99 | 50.63 | 46.02 |
| IVOMD, % | 64.13 | 65.38 | 64.17 | 65.47 | 65.66 | 65.18 |

Out of Tables 1 and 2, it can be concluded that

- With regards to the total VFA, all samples comprising urea, biuret and nitrates (UBN 1, 2 and 3) have a higher amount in comparison with UBN 0 and in comparison with the control without NPN supplementation or with urea supplementation. These findings indicate that UBN 1, 2, and 3 are more effective than the other NPN sources tested herein, i.e. urea and a mixture of urea and biuret (UBN 0) in supporting in vitro fermentation.
- With regards to ammonia-N, as expected, the supplementation with NPN, i.e. urea, UBN 0, 1, 2, and 3, increased its concentration.
- With regards to IVDMD, supplementation of control with UBN sources, but not with urea, improved the extent of dry matter degradation. This effect was most pronounced for UBN 1 and UBN 2. In particular, supplementation with UBN 1 resulted in an increased IVDMD by about 9%, compared to the control without NPN or with urea as NPN, and in an increased IVDMD by about 7% compared to a urea/biuret mixture as NPN (i.e. UBN 0). Supplementation with UBN 2 resulted in an increased IVDMD by about 12%, compared to the control without NPN or with urea as NPN, and in an increased IVDMD by about 10% compared to a urea/biuret mixture as NPN (i.e. UBN 0). These findings further support the proposition that supplementation with UBN, in particular UBN 1 or UBN 2, is more effective in supporting rumen fermentation than Urea.
- With regards to IVOMD, results further confirm that UBN 1 and UBN 2 were the most effective source of NPN in supporting substrate fermentation by increasing OM degradation to larger extent than urea and other UBN sources.

Overall, these results demonstrate that a mixture of urea, biuret and one or more nitrate compounds, such as a particularly UBN is an effective source of NPN that outperforms urea and a urea / biuret mixture (UBN 0) in supporting in vitro fermentation.

In Table 3, the concentration of bacterial N, and the bacteria efficiency (BE) or, stated differently, the efficiency of microbial protein synthesis (EMPS), i.e. g of bacteria N / kg of OM truly digested, for (i) a control diet without NPN supplementation (Control), (ii) a corn silage-based diet with a urea supplementation as NPN (Urea) or (iii) a corn silage-based diet with supplementation with different urea-biuret-nitrate mixtures ("UBN"), i.e. UBN 0, UBN 1, UBN 2, UBN 3, with a ratio of urea+ biuret versus nitrates of 100 : 0; 75 : 25; 50 : 50; and 25 : 75 respectively, after 24 hours of incubation is shown. These parameters define the capacity of rumen microbes to use N.

*Table 3: Effect of NPN supplementation on microbial synthesis and microbial efficiency production in a corn-based diet*

| Variable | Control | Urea | UBN mixtures | | | |
|---|---|---|---|---|---|---|
| | | | UBN 0 100 : 0 | UBN 1 75 : 25 | UBN 2 50 : 50 | UBN 3 25 : 75 |
| Bacteria N, mg | 55.66 | 67.34 | 59.55 | 62.58 | 68.70 | 64.68 |
| EMPS | 27.07 | 34.46 | 41.98 | 35.18 | 37.39 | 36.12 |

Out of Table 3, it can be concluded that the supplementation of the control diet with NPN increases the amount of N assimilated by bacteria. This demonstrates that methane production is not reduced at the expense of reducing microbial growth and fermentation.

There is consequently a positive impact, i.e. an increase, on the assimilation of bacteria N, particularly compared to urea as NPN.

**Methane production**

When feed is eaten by a ruminant, the nutrients are initially in the form of carbohydrates, proteins and fats (or lipids). During the fermentation of the carbohydrates, carbon dioxide and methane are produced.

The amount of methane produced was determined by measuring total gas production and the contribution of methane to it. The production of gas was recorded using an Ankom Gas Monitoring System from Ankom Technologies. This system was adapted to 500 ml bottles during the entire fermentation process. The kinetics of the production was fitted to a Gompertz model to determine the rate of the total maximal gas production (M), fractional rate of gas produced (kf) and lag phase (L). A gas subsample was analyzed to measure methane concentration and calculate the total methane production during the fermentation. The concentration of methane was determined using a gas chromatograph Agilent 7820 GC from Agilent Technologies with flame ionization and a capillary column (Plot Fused Silica 25 m x 0.32 mm, coating Molsieve 5A, Varian CP 7536 from Varian Inc.).

In Table 4, the effect of NPN supplementation on the production of methane in a corn silage-based diet is shown. the effect of NPN supplementation on the production of methane in a corn silage-based diet is shown. More in particular, methane gas production expressed in mM/g organic matter incubated (OMi) and expressed in mM/g organic matter digested (OMd), in a control diet without NPN supplementation ("control"), a corn silage-based diet either urea supplementation ("urea") or a corn silage-based diet with supplementation with different urea-biuret-nitrate mixtures ("UBN"), i.e. UBN 0, UBN 1, UBN 2, UBN 3, with a ratio of urea+ biuret versus nitrates of 100 : 0; 75 : 25; 50 : 50; and 25 : 75, respectively, are shown.

*Table 4: Effect of NPN supplementation on methane production in a corn silage-based diet*

| Variable | Control | Urea | UBN mixtures | | | |
|---|---|---|---|---|---|---|
| | | | UBN 0 100 : 0 | UBN 1 75 : 25 | UBN 2 50 : 50 | UBN 3 25 : 75 |
| m$M$/g OMi | 1.24 | 1.23 | 1.18 | 1.16 | 1.12 | 1.07 |
| m$M$/g OMd | 6.93 | 6.86 | 6.58 | 6.26 | 6.25 | 5.96 |

Out of Table 4, the production of methane per g of OMi or OMd decreased as the proportion of nitrates increased in UBN (amount of mM/g OMi and amount of mM/g OMd for UBN 0 > for UBN 1 > UBN 2 > UBN3).

More in particular, supplementation with UBN 1 resulted in decrease in methane production by about 6% (expressed per g OMi) or by about 10% (expressed per g OMd), compared to the control without NPN, and by about 6% (expressed per g OMi) of by about 9% (expressed per g OMd), compared to the diet with urea as NPN.

Supplementation with UBN 2 resulted in decrease in methane production by about 10% (expressed per g OMi) or by about 10% (expressed per g OMd), compared to the control without NPN, and by about 9% (expressed per g OMi) of by about 9% (expressed per g OMd), compared to the diet with urea as NPN.

Therefore, there is a positive impact, i.e. a reduction, on the methane production by the combination of urea, biuret and nitrate as an NPN source.

Overall, UBN 1 as NPN-source particularly combined a significantly increased methane reduction with a greatly increased *in vitro* dry matter and organic matter digestibility and increased amount of bacteria nitrogen produced per kg organic matter digested, compared to urea as NPN source.

*EXAMPLE 2:*

*Animals, experimental design and treatments*

A total of 72 crossbred yearling steers [303 kg $\pm$ 29 kg initial body weight (BW) were stratified by body weight (BW) and randomly allocated to three treatments. All steers consumed a basal diet of corn silage, cotton-gin trash, and a premix of vitamins and minerals.

The steers were housed in pens (8 steers / pen, 3 pens / treatment). The steers were supplemented with three different NPN-sources on an isonitrogenous basis, considering as reference the N provided by urea when included at 1 wt.% of the diet in dry matter (DM-basis.

In Table 5, the composition of the basal diet supplemented with three different types of NPN, i.e. urea (Urea) (46 % N, Yara International), a mixture of urea and biuret (UB) (41 % N) and a mixture of urea, biuret and nitrate according to the present disclosure ("UBN") (35 % N, 21.3 % $NO_3$) is shown.

In order to determine DM of the feed, approximately 0.5 g of sample was weighed in duplicate, dried in a forced-air oven at 100 °C for 24 hours, and ashed at 550 °C for six hours.

For the determination of the fibrous component, samples were weighed in duplicate into F56 bags (Ankom Technology Crop., Macedon, NY) and analyzed for neutral detergent fiber (NDF), using heat-stable $\alpha$-amylase and sodium sulfite, and subsequently for acid detergent fiber (ADF) as described by Van Soest et al. (1991) in Ankom 200 Fiber Analyzer (Ankom Technology Corp., Macedon, NY).

The concentration of N was analyzed through the Dumas dry combustion method using a Vario Micro Cube (Elementar, Manchester, UK) after samples were ball-milled using a Mixer Mill MM 400 (Retsch) at 25 Hz for 9 minutes.

The feed samples were analyzed for concentration of starch through the enzymatic-colorimetric method of Hall (2015) with the following modifications: glucose was analyzed using a quantitative colorimetric kit (G7521-11, Pointe Scientific Inc., Canton, MI), absorbance was read on 200 $\mu$l samples at OD520 in flat-bottom 96-well plates (Corning Costar 3361, Thermo Fisher Scientific Inc., Waltham, MA) using a plate reader (Fisherbrand UV / VIS AccuSkan GO Spectrophotometer, Thermoe Fisher Scientific Inc., Hampton, NH).

*Table 5: Composition of the basal diet supplemented with different types of NPN*

| Item | Treatment | | |
|---|---|---|---|
| | U | UB | UBN |
| Ingredient, wt.% of dry matter | | | |
| Corn silage | 77.00 | 76.88 | 76.69 |
| Cotton-gin trash | 20.00 | 20.00 | 20.00 |
| Mineral | 2.00 | 2.00 | 2.00 |
| NPN source | 1.00 | 1.12 | 1.31 |
| Composition | | | |
| DM (dry matter), wt.% as fed | 37.13 | 36.17 | 36.97 |
| CP (crude protein), wt. % of DM | 13.20 | 12.70 | 12.90 |
| Soluble CP, wt. % of CP | 49.00 | 53.00 | 55.00 |
| iNDF (indigestible neutral detergent fiber) | 41.40 | 38.30 | 39.00 |
| ADF (acid detergent fiber) | 30.10 | 29.40 | 31.00 |
| Starch, wt.% of DM | 27.70 | 29.80 | 27.20 |
| Ash, wt.% of DM | 5.91 | 5.78 | 6.34 |

*Animal adaptation*

From day -17 to day -16 relative to the beginning of the experimental period, the steers received 20 % of their total supplemental NPN.

From day -15 to day -12 relative to the beginning of the experimental period, the steers received 40 % of their total supplemental NPN.

From day -11 to day -8 relative to the beginning of the experimental period, the steers received 60% of their total supplemental NPN.

From day -7 to day -3 relative to the beginning of the experimental period, the steers received 80 % of their total supplemental NPN.

From day -2 to the beginning of the experimental period (day 0), the steers received 100% of their total supplemental NPN.

### *Blood profile measurements*

Blood samples were collected every 14 days during the performance evaluation via jugular venipuncture into 10 ml evacuated tubes containing Na heparin (BD Vacutainer, Franklin Lakes, NJ) and placed on ice centrifuged at 1,500 x g for 15 minutes at 4 °C. Plasma was transferred to polypropylene tubes (12 x 75 mm, Fisherbrand, Thermo Fisher Scientific Inc, Waltham, MA) and stored at - 20 °C for further analysis.

### *Enteric methane emission measurements*

When feed is eaten by a ruminant, the nutrients are initially in the form of carbohydrates, proteins and fats (or lipids). During the fermentation of the carbohydrates, carbon dioxide and methane are produced.

Methane emission weas measured in all steers for five consecutive days using the $SF_6$ tracer technique (Johnson et al., 1994; Henry et al., 2020). In this technique, the steers received a calibrated permeation tube filled with $SF_6$ (3.1 g $\pm$ 0.2 g) before the measurement period.

The average $SF_6$ release rate was 5.2 g $\pm$ 1.1 mg/d. After five days of training, a vacuum gas collector canister was attached to each animal to collect a sample of daily gas production. Each day at the same hour, a new vacuum canister was replaced throughout the five days of the measurement period. Five collection canisters were used to determine environmental $CH_4$ and $SF_6$ concentrations. A gas sample was taken from each canister and conserved in 120 ml bottles for posterior analysis of $CH_4$ and $SF_6$ using gas chromatography. For the current experiment, only steers with a least three successful days of collection and measurement were considered in the final analysis. Gas samples were analyzed by gas chromatography (Agilent 7820A GC, Agilent Technologies, Palo Alto, CA). A flame ionization and electron capture detector were used for $CH_4$ and $SF_6$ analysis, respectively, with a capillary column (Plot Fused Silica 25 m x 0.32 mm, Coating Molsieve 5A, Varian CP7536, Varian Inc., Lake Forest, CA). The injector, column and detector temperatures for $CH_4$ analysis were 80, 160 and 200 °C, respectively. For $SF_6$, temperatures were 50, 30 and 300 °C for the injector, the column and the detector, respectively. The carrier gas for $CH_4$ and $SF_6$ was $N_2$. The emissions of $CH_4$ were calculated according to the next equation:

$$QCH_4 = QSF_6 \ x \frac{([CH_4]\gamma - [CH_4]\beta)}{([SF_6]\gamma - [SF_6]\beta)}$$

$QCH_4$ = $CH_4$ emissions per animal (g / day)
$QSF_6$ = $SF_6$ release rate (mg / day)
$[CH_4]\gamma$ = the concentration of $CH_4$ in the animals' collection canister
$[CH_4]\beta$ = the concentration of $CH_4$ in the environmental collection canister
$[SF_6]\gamma$ = the concentration of $SF_6$ in the animals' collection canister
$[SF_6]\beta$ = the concentration of $SF_6$ in the environmental collection canister

### *Gain to feed ratio*

The gain to feed ratio (G:F) was computed as the ratio of the average daily gain (ADG) to daily dry matter intake (DMI). The feed intake was recorded daily. The individual intake was determined via the GrowSafe® intake monitoring system. In cattle management, GrowSafe Systems is a commonly used tool to closely monitor individual animal feeding data using radio frequency identification (RFID) tag technology. The animals are equipped with a unique RFID tag that is read by the feed bunks each time the animal lowers its head into the bunk to consume feed. Average daily weight gain (ADG) and feed efficiency were calculated during the performance evaluation. The ADG was determined by the difference between the final BW and the initial BW divided by the number of days of the performance evaluation (56 days). The initial BW was calculated as the average unshrunk BW of heifers on days 16 and 17. The final BW was the average unshrunk BW measured on days 72 and 73. The unshrunk BW was obtained every 14 days.

*Effect of NPN supplementation on animal performance and plasma urea nitrogen (PUN)*

In Table 6, the effect of NPN supplementation with Urea, UB and UBN on growth performance is shown. More in particular, the initial and the final BW and the ADG, all expressed in kg, the dry matter intake (DMI) expressed in kg / day and in percentage of BW, the gain to feed ratio (G:F) expressed in kg / kg, the net energy of gain (NEg) expressed in Mcal / kg of DM and the plasma urea nitrogen (PUN) expressed in m / dL are shown.

*Table 6: Effect of NPN supplementation on animal performance and the plasma urea nitrogen (PUN)*

| Item | Treatment | | |
|---|---|---|---|
| | +Urea | +UB | +UBN |
| Initial BW (kg) | 288.69 | 283.18 | 289.92 |
| Final BW (kg) | 343.69 | 342.11 | 349.48 |
| ADG (kg) | | | |
| 0 - 14 | 0.78 | 0.72 | 0.75 |
| 0-28 | 0.81 | 0.72 | 0.96 |
| 0-42 | 1.02 | 1.04 | 1.12 |
| 0-56 | 1.01 | 1.05 | 1.05 |
| DMI (kg / day) | | | |
| 0 - 14 | 6.82 | 6.91 | 6.89 |
| 0-28 | 7.03 | 7.40 | 7.29 |
| 0-42 | 7.24 | 7.85 | 7.55 |
| 0 - 56 | 7.29 | 7.98 | 7.52 |
| DMI (% of BW) | 2.31 | 2.54 | 2.34 |
| G:F (kg / kg) | | | |
| 0 - 14 | 0.10 | 0.10 | 0.10 |
| 0-28 | 0.11 | 0.10 | 0.13 |
| 0-42 | 0.14 | 0.13 | 0.15 |
| 0-56 | 0.14 | 0.13 | 0.14 |
| NEg (Mcal / kg of DM) | 0.55 | 0.52 | 0.55 |
| PUN (mg / dL) | | | |
| 0 | 7.80 | 7.59 | 6.81 |
| 14 | 6.76 | 7.52 | 7.18 |
| 28 | 10.41 | 8.78 | 7.73 |
| 42 | 9.25 | 7.55 | 7.65 |
| 56 | 8.20 | 8.60 | 8.01 |

Out of Table 6, it can be concluded that

- the supplementation with UBN, a NPN composition according to the present disclosure, showed the greatest ADG compared with the supplementation with Urea and UB, particularly during the first 28 days, with an about 18% higher and about 33% higher ADG, compared to a diet containing urea or a urea/biuret mixture, respectively, as NPN source.
- the G:F ratio was the greatest for UBN during the first 28 days.

*Effect of NPN supplementation on enteric emissions of beef steers*

Methane is an energy loss representing between 2 % and 12 % of the gross energy intake in ruminants. Nitrates reduction is energetically more efficient than $CH_4$ synthesis and competes for $H_2$ during the ruminal fermentation, and nitrates formed during the nitrate reduction affect some bacteria and methanogen population resulting in lower $CH_4$ emissions. The $CH_4$ reduction can thus improve animal performance because of the reallocation of the energy into productive fates like protein accretion. In this regard, it has been reported that there was a greater energy retention when animals received nitrates in the diet.

In Table 7, the effect of the NPN supplementation with Urea, UB and UBN on enteric methane emission of beef steers is shown. More in particular, the intake expressed in kg/day, and the methane emission expressed in g/kg DMI, g/kg OMI and g/kg ADG are shown.

Table 7: Effect of NPN supplementation on enteric methane emission of beef steers

| Item | Treatment | | |
|---|---|---|---|
| | U | UB | UBN |
| DM intake (kg / day) | 6.08 | 7.11 | 6.30 |
| Methane emission | | | |
| (g / kg DMI) | 36.59 | 31.21 | 33.93 |
| (g / kg OMI) | 38.68 | 32.94 | 36.02 |
| (g / kg ADG) | 213.79 | 212.31 | 206.52 |

Out of Table 7, it can be concluded that supplementing the diet of yearling steers with UB or UBN reduced methane emission without compromising or even improving their performance. Compared to supplementation with urea, supplementation with UBN reduced the methane emissions by about 7%, when expressed as g/kg DMI or g/kg OMI.

In this context, taking into account that the UBN is included in the diet at a concentration of 1.31wt% (see table 5), this corresponds to a nitrate inclusion in the diet of about 0.27 wt%. The expected reduction in methane production/emissions would be about 2.7%. The observed reduction of about 7% in methane production is thus clearly greater than expected, indicating the composition according to the present disclosure, comprising urea, biuret and one or more nitrate compound has a synergistic effect on enteric methane emissions.

### EXAMPLE 3 - Increasing doses of UBN on fermentation and methane production in vitro

The main objective of example 3 was to assess the impact of increasing doses of a NPN composition according to the present disclosure, comprising urea, biuret and one or more nitrate compounds, hereinafter UBN, on *in vitro* fermentation, nutrient digestibility, and methane production in a corn silage-based diet.

### Approach and Research Procedures

Two ruminally cannulated steers were used as ruminal fluid donors for the *in vitro* batch culture incubations. The steers were fed corn silage, cotton gin trash, and a premix of vitamins and minerals (70, 28, and 2 % on a dry matter basis, respectively) ad libitum at least 35 d before collecting ruminal fluid to perform the in vitro incubations. To adapt steers to the presence of NPN, each steer received the N equivalent to 100 g of urea per day (46 g of N daily/steer) but divided into the two NPN sources. Thus, each steer fed daily 33 g of urea, or 43 g of a UBN mixture according to the present disclosure. These amounts were mixed in with the diet daily. The nitrates in the NPN source was gradually introduced to the diet as follows: 30% of total maximum during the first week (13 g/animal/day), 60% of the total maximum during the second week (26 g/animal/d), and 100% in the last week (43 g/animal/d). *In vitro* incubations were conducted on three separate days (replicates) using the same silage fed to the steers as the incubation substrate. The sources of NPN to be tested were analyzed for total N content and incubated on an isonitrogenous basis.

### Treatments

The incubation substrates (treatments) to test were control (CON, without NPN supplementation) and four increasing inclusions (from 1× to 4×) of the Urea-Biuret-Nitrate mixture (UBN) according to the present disclosure. Treatments at 1× were designed to be iso-nitrogenous and equivalent to 1% of the inclusion in dry matter basis of urea (Yara International), in the diet, except for the CON treatment.

*Batch culture incubations*

*In vitro* incubations were conducted on three separate days (replicates) using the same silage fed to the steers as the incubation substrate. A representative sample of rumen digesta was collected from different places in the rumen from the ruminally-cannulated steers and strained through 4 layers of cheesecloth, placed in pre-warmed thermos containers, and transported to the laboratory within 30 min of collection. In the laboratory, ruminal fluid from the two steers was maintained under constant $CO_2$ flux and was combined in equal proportions. Combined ruminal fluid was mixed with McDougall buffer in relation 1:4 (i.e., inoculum).

Two bottles of 120 mL per treatment containing 0.7 g and 50 mL of inoculum were incubated for 24 h at 39°C with gentle agitation (60 rpm). Further, two bottles without substrate were incubated as blank bottles. Gas pressure was recorded at 24 h, and a sample of gas was stored for further analysis. At the end of the 24 h of incubation, the final pH of fermentation fluid was recorded, and two 10-mL samples were collected and acidified by adding 20 μL of a 20% (vol/vol) of $H_2SO_4$ solution and were frozen at -20°C until further analysis.

Measurement of *in vitro* organic matter digestibility (IVOMD) was performed by incubating a separate set of duplicate 100-mL polypropylene tubes for 24 h at 39°C on each of the replicate days. These flasks contained 0.7 g of the substrate and were inoculated with 50 mL of a 4:1 McDougall's buffer:ruminal fluid mixture. After 24-h incubation with ruminal fluid, a pepsin-HCl solution was added, and tubes were incubated for another 48 h before filtering, drying at 105°C in a forced air oven for 24 h, and ashing at 550°C for 6 h to determine the undigested organic matter.

*Sample analysis*

Concentrations of $NH_3$-N in the incubation fluid were measured after centrifuging at 10,000 x g for 15 min at 4°C (Avanti J-E, Beckman Coulter Inc., Palo Alto, CA) following the methodology described by Broderick and Kang (1980). Briefly, 1 mL of a phenol reagent was pipetted into $12 \times 75$ mm borosilicate disposable culture tubes (Fisherbrand; Thermo Fisher Scientific Inc., Waltham, MA). A 20-μL aliquot of the supernatant from the centrifuged sample was transferred to the phenol-containing culture tubes. After vortexing, 0.8 mL of a hypochlorite solution was added to the mixture and vortexed again. The culture tubes were covered with glass marbles and placed in a water bath at 95°C for 5 min. Absorbance was read on 200 μL samples at $OD_{620}$ in flat-bottom 96-well plates (Corning Costar 3361, Thermo Fisher Scientific Inc., Waltham, MA) using a plate reader (Fisherbrand UV/VIS AccuSkan GO Spectrophotometer, Thermo Fisher Scientific Inc., Hampton, NH). All assays were conducted in duplicate determinations with subsequent analyses performed when CVs were above 5%.

Total gas production was measured using a manual transducer (Digital Test Gauge, Ashcroft Inc., Stratford, CT, USA), and a subsample was analyzed to measure $CH_4$ concentration by gas chromatography to calculate total $CH_4$ production during the course of the fermentation. To determine concentrations of $CH_4$, a gas chromatograph (Agilent 7820A GC, Agilent Technologies, Palo Alto, CA) using flame ionization and a capillary column (Plot Fused Silica 25 m $\times$ 0.32 mm, Coating Molsieve 5A, Varian CP7536; Varian Inc., Lake Forest, CA) was used. Temperatures of the injector, column, and detector were 80, 160, and 200°C, respectively, and $N_2$ was the carrier gas flowing at 3.3 mL/min. The split ratio for the injected $CH_4$ sample was 100:1.

Additionally, total volatile fatty acids (VFA) concentrations were measured by gas chromatography in the incubation fluid at the end of the fermentation to assess potential changes in the profile of VFA being produced, following the methodology described by Ruiz-Moreno et al. (2015). Briefly, samples were centrifuged at 10,000 $\times$ g for 15 min at 4°C. Two milliliters of the supernatant were mixed with 0.4 mL (5:1 ratio) of a metaphosphoric:crotonic acid (internal standard) solution, and samples were frozen overnight. Samples were thawed and centrifuged again at 10,000 $\times$ g for 15 min at 4°C. The supernatant was transferred into 12 mm $\times$ 75 mm borosilicate disposable culture tubes (Fisherbrand; Thermo Fisher Scientific Inc., Waltham, MA) and mixed with ethyl acetate to form a 2:1 ethyl acetate: supernatant mixture. Culture tubes were vigorously shaken and followed by a 5 min rest time to allow the separation of the ethyl acetate. A subsample of the ethyl acetate was transferred into small vials prior to analysis. Samples were analyzed with a gas chromatograph (Agilent 7820A GC, Agilent Technologies) using a flame ionization detector and a capillary column (CP-WAX 58 FFAP 25 m $\times$ 0.53 mm, Varian CP7767; Varian Inc.). The column temperature was maintained at 110°C, and the injector and detector temperatures were 200 and 220°C, respectively.

*RESULTS - Incresing dose of UBN on in vitro fermentation and $CH_4$ emissions*

The results are listed in Table 8

Table 8. Effect of increasing the inclusion of a urea-biuret-nitrate mixture according to an embodiment of the present disclosure (UBN) on fluid fermentation pH, the concentrations of volatile fatty acids and ammonia-N, in vitro organic matter digestibility, and gas and methane production in a corn silage-base diet.

| Variable | UBN mixture | | | | |
|---|---|---|---|---|---|
| | 0 | 1x | 2x | 3x | 4x |
| pH | 6.5 | 6.56 | 6.54 | 6.57 | 6.63 |
| Total volatile fatty acids, m$M$ | 88.72 | 101.69 | 103.86 | 107.83 | 108.17 |
| Acetate, m$M$ | 55.57 | 63.04 | 64.58 | 68.36 | 68.94 |
| Propionate, m$M$ | 18.19 | 21.46 | 22.33 | 22.59 | 22.89 |
| Butyrate, m$M$ | 10.79 | 12.46 | 12.32 | 12.26 | 11.9 |
| Acetate, mol/100 mol | 62.64 | 61.95 | 62.15 | 63.33 | 63.71 |
| Propionate, mol/100 mol | 20.52 | 21.13 | 21.56 | 20.93 | 21.11 |
| Butyrate, mol/100 mol | 12.15 | 12.28 | 11.83 | 11.45 | 11.07 |
| Acetate: Propionate | 3.05 | 2.95 | 2.9 | 3.03 | 3.02 |
| Ammonia-N, mM | 3.25 | 6.71 | 10.77 | 13.37 | 15.98 |
| IVOMD, % | 54.4 | 58.33 | 63.75 | 62.83 | 58.28 |
| Gas production, mL/g OM d | 300.26 | 270.52 | 236.28 | 240.73 | 251.76 |
| Methane production, m$M$/g OMd | 33.73 | 26.66 | 23.95 | 23.29 | 22.11 |
| [1] IVOMD: In vitro organic matter digestibility; OMd: Organic matter degraded. | | | | | |

The increasing doses of UBN linearly increased the concentration of $NH_3$-N, the production of total VFA, acetate (expressed in mM), and propionate (expressed in mM), and the rumen fluid pH. Additionally, the IVOMD and the concentration of acetate (mol/100mol) showed a quadratical increase when increasing UBN inclusion. Also, increasing the inclusion of UBN lineally reduces $CH_4$ production, with a reduction of about 20% of methane production (expressed as mM/g OMd) for the 1x UBN dose (compared to the control without NPN supplementation), and a reduction of about 28%-35% for the 2x, 3x and 4x UBN dose.

For other parameters, the trend was not uniform. For instance, the acetate:propionate ratio was reduced upon incorporation of the UBN mixture, and this effect was the greatest at the 1x and 2x doses, but lower for the 3x and 4x doses.

In the present experiment, the inclusion of UBN increased the $NH_3$-N concentration resulting in greater IVOMD and concentration of total and individual VFA (Table 8), indicative of a greater fermentation. Rumen microbes can utilize $NH_3$-N to synthezise microbial protein promoting greater fermentation. Additionally, the greater concentration of $NH_3$-N is in line with the linear increase in the rumen fluid pH.

Inclusion of UBN reduced the acetate: propionate ratio, particularly at the 1x and 2x doses. However, an increasing inclusion of UBN promoted greater fermentation towards promoting acetate synthesis. Without wishing to be bound by theory, nitrates can capture $H_2$ during its reduction to $NH_3$, resulting in lower $H_2$ pressure and promoting acetate synthesis, resulting in a greater acetate proportion at the 3x and 4x doses compared to the 1x and 2x doses (Table 8).

Increasing UBN inclusion linearly reduced $CH_4$ production. Without wishing to be bound by theory, nitrate supplementation reduces $CH_4$ emissions because nitrate reduction competes with methanogenesis, thereby reducing the $H_2$ available to reduce $CO_2$ to $CH_4$. Also, some intermediate compounds produced during the nitrate reduction may be toxic for methanogenic microorganisms resulting in lower $CH_4$ production. Possibly the greater concentration of $NH_3$-N during the incubation results in a greater fermentation rate, increasing the concentration of VFA and IVOMD.

Claims

1. A ruminant feed supplement composition comprising between 5 and 60 wt% of urea, between 2 and 60 wt% of biuret and between 20 and 40 wt% of one or more nitrate compounds, based on the total weight of the composition.

2. The ruminant feed supplement composition according to claim 1, wherein the composition comprises between 20 and 40 wt% of urea, between 20 and 40 wt% of biuret, and between 21 and 37 wt% of one or more nitrate compounds, based on the total weight of the composition; more in particular wherein the composition comprises between 25 and 35 wt.% of urea, between 25 and 35 wt.% of biuret and between 23.5 and 31.5 wt.% of one or more nitrate compounds, based on the total weight of the composition.

3. The ruminant feed supplement composition according to claim 1 or 2, wherein the nitrate compound is a water-soluble nitrate salt, more in particular chosen out of calcium nitrate, magnesium nitrate, ammonium nitrate, potassium nitrate and/or sodium nitrate, and most in particular comprising between 20 and 32 wt% of calcium nitrate and between 1 and 5 wt% of ammonium nitrate, based on the total weight of the composition.

4. The ruminant feed supplement composition according to any one of claims 1 to 3, wherein the composition further comprises between 1 and 5 wt.% of triuret, between 0.5 and 3 wt.% of ammelide, between 3 and 30 wt.% of cyanuric acid, and/or between 0.1 and 4.5 wt.% of moisture, with wt% based on the total weight of the composition.

5. Use of a ruminant feed supplement composition comprising between 5 and 60 wt% of urea, between 2 and 60 wt% of biuret and between 20 and 40 wt% of one or more nitrate compounds, based on the total weight of the composition, as a dietary non-protein nitrogen source for ruminants, and for simultaneously decreasing the enteric methane production of a ruminant animal, and more in particular further comprising between 1 and 5 wt.% of triuret, between 0.5 and 3 wt.% of ammelide, between 3 and 30 wt.% of cyanuric acid, and/or between 0.1 and 4.5 wt.% of moisture, with wt% based on the total weight of the composition.

6. Use according to claim 5, for further:

   - improving the efficiency of microbial protein synthesis (EMPS) by rumen microbes in a ruminant animal; and/or
   - decreasing the acetate to propionate ratio produced by rumen microbes in a ruminant animal; and/or
   - increasing the dry matter (DM) and and organic matter (OM) digestibility; and/or
   - for improving the average daily gain (ADG) of a ruminant animal; and/or
   - for improving the feed efficiency of a ruminant animal.

7. Use according to claim 5 or 6, wherein the composition comprises between 20 and 40 wt% of urea, between 20 and 40 wt% of biuret, and between 21 and 37 wt% of one or more nitrate compounds, based on the total weight of the composition; particularly, wherein the composition comprises between 25 and 35 wt.% of urea, between 25 and 35 wt.% of biuret and between 23.5 and 31.5 wt.% of one or more nitrate compounds, based on the total weight of the composition, more in particular wherein the nitrate compound is a water-soluble nitrate salt, and most in particular chosen out of calcium nitrate, magnesium nitrate, ammonium nitrate, potassium nitrate and/or sodium nitrate.

8. Use according to claim 7, wherein the composition comprises between 20 and 32 wt% of calcium nitrate and between 1 and 5 wt% of ammonium nitrate, based on the total weight of the composition.

9. Use according to any one of claims 5 to 8, wherein the composition is a ruminant feed supplement composition according to any one of claims 1 to 4.

10. A method for simultaneously reducing the enteric methane emissions of a ruminant animal and for providing a dietary non-protein nitrogen source, comprising administering to the ruminant animal a ruminant feed supplement composition as a NPN source, wherein the ruminant feed supplement composition comprises between 5 and 60 wt% of urea, between 2 and 60 wt% of biuret and between 20 and 40 wt% of one or more nitrate compounds, based on the total weight of the composition, and optionally comprising the steps of preparing or providing a ruminant feed composition comprising the ruminant feed supplement composition as a NPN source before administering the ruminant feed composition to the ruminant animal.

11. The method according to claim 10, wherein the enteric methane emissions of the ruminant animal, expressed per kg dry matter intake or per kg organic matter intake is reduced by at least 5% when the ruminant feed supplement composition is administered to a ruminant as NPN source compared to a diet comprising urea as NPN source.

12. The method according to claim 10 or 11, wherein said method is further simultaneously a method for improving the average daily gain (ADG) of a ruminant animal, in particular wherein the ADG of the ruminant animal is increased by at least 5%, more in particular by at least 10%, or even more in particular by at least 15%, when the ruminant feed supplement composition is administered to a ruminant as NPN source compared to a diet comprising urea as NPN source, and, optionally, wherein said method is further simultaneously a method for

- improving the efficiency of microbial protein synthesis (EMPS) by rumen microbes in a ruminant animal; and/or
- decreasing the acetate to propionate ratio produced by rumen microbes in a ruminant animal; and/or
- increasing the dry matter (DM) and organic matter (OM) digestibility; and/or
- improving the feed efficiency of a ruminant animal.

13. The method according to any one of claims 10 to 12, wherein the ruminant feed supplement composition comprises between 20 and 40 wt% of urea, between 20 and 40 wt% of biuret, and between 21 and 37 wt% of one or more nitrate compounds, based on the total weight of the composition; particularly, wherein the ruminant feed supplement composition as a NPN source comprises between 25 and 35 wt.% of urea, between 25 and 35 wt.% of biuret and between 23.5 and 31.5 wt.% of one or more nitrate compounds, based on the total weight of the composition, more in particular wherein the nitrate compound is a water-soluble nitrate salt, even more particularly wherein the water-soluble nitrate salt is chosen out of calcium nitrate, magnesium nitrate, ammonium nitrate, potassium nitrate and/or sodium nitrate, most in particular wherein the ruminant feed supplement composition comprises between 20 and 32 wt% of calcium nitrate and between 1 and 5 wt% of ammonium nitrate, based on the total weight of the composition.

14. The method according to any one of claims 10 to 13, wherein the ruminant feed supplement composition further comprises between 1 and 5 wt.% of triuret, between 0.5 and 3 wt.% of ammelide, between 3 and 30 wt.% of cyanuric acid, and/or between 0.1 and 4.5 wt.% of moisture, with wt% based on the total weight of the composition.

15. The method according to any one of claims 10 to 14, wherein the ruminant feed supplement composition is present in the ruminant feed composition in a concentration between 0.5 and 4 wt.%, more in particular between 0.5 and 3.5 wt.% or between 0.5 and 3 wt.%, even more in particular between 1 and 2.5 wt.%, with wt% based on the total dry matter basis of the ruminant feed composition.

## Patentansprüche

1. Wiederkäuerfutterergänzungszusammensetzung, die zwischen 5 und 60 Gew.-% Harnstoff, zwischen 2 und 60 Gew.-% Biuret und zwischen 20 und 40 Gew.-% einer oder mehrerer Nitratverbindungen, bezogen auf das Gesamtgewicht der Zusammensetzung, umfasst.

2. Wiederkäuerfutterergänzungszusammensetzung nach Anspruch 1, wobei die Zusammensetzung zwischen 20 und 40 Gew.-% Harnstoff, zwischen 20 und 40 Gew.-% Biuret und zwischen 21 und 37 Gew.-% einer oder mehrerer Nitratverbindungen, bezogen auf das Gesamtgewicht der Zusammensetzung, umfasst; wobei die Zusammensetzung insbesondere zwischen 25 und 35 Gew.-% Harnstoff, zwischen 25 und 35 Gew.-% Biuret und zwischen 23,5 und 31,5 Gew.-% einer oder mehrerer Nitratverbindungen, bezogen auf das Gesamtgewicht der Zusammensetzung, umfasst.

3. Wiederkäuerfutterergänzungszusammensetzung nach Anspruch 1 oder 2, wobei es sich bei der Nitratverbindung um ein wasserlösliches Nitratsalz handelt, insbesondere ausgewählt aus Calciumnitrat, Magnesiumnitrat, Ammoniumnitrat, Kaliumnitrat und/oder Natriumnitrat, und wobei die Zusammensetzung ganz insbesondere zwischen 20 und 32 Gew.-% Calciumnitrat und zwischen 1 und 5 Gew.-% Ammoniumnitrat, bezogen auf das Gesamtgewicht der Zusammensetzung, umfasst.

4. Wiederkäuerfutterergänzungszusammensetzung nach einem der Ansprüche 1 bis 3, wobei die Zusammensetzung weiterhin zwischen 1 und 5 Gew.-% Triuret, zwischen 0,5 und 3 Gew.-% Ammelid, zwischen 3 und 30 Gew.-% Cyanursäure und/oder zwischen 0,1 und 4,5 Gew.-% Feuchtigkeit umfasst, wobei Gew.-% auf das Gesamtgewicht der Zusammensetzung bezogen sind.

5. Verwendung einer Wiederkäuerfutterergänzungszusammensetzung, die zwischen 5 und 60 Gew.-% Harnstoff, zwischen 2 und 60 Gew.-% Biuret und zwischen 20 und 40 Gew.-% einer oder mehrerer Nitratverbindungen, bezogen auf das Gesamtgewicht der Zusammensetzung, umfasst, als Nahrungs-Nichtproteinstickstoffquelle für Wiederkäuer und zur gleichzeitigen Verringerung der enterischen Methanproduktion eines Wiederkäuers, und insbesondere weiterhin zwischen 1 und 5 Gew.-% Triuret, zwischen 0,5 und 3 Gew.-% Ammelid, zwischen 3 und 30 Gew.-% Cyanursäure und/oder zwischen 0,1 und 4,5 Gew.-% Feuchtigkeit, wobei Gew.-% auf das Gesamtgewicht der Zusammensetzung bezogen sind, umfasst.

6. Verwendung nach Anspruch 5, weiterhin zum:

- Verbessern der Effizienz der mikrobiellen Proteinsynthese (EMPS) durch Pansenmikroben bei Wiederkäuern; und/oder

- Verringern des durch Pansenmikroben bei einem Wiederkäuer erzeugten Acetat-Propionat-Verhältnisses; und/oder

- Erhöhen der Verdaulichkeit der Trockenmasse (Dry Matter, DM) und der organischen Substanz (Organic Matter, OM); und/oder

- Verbessern der durchschnittlichen täglichen Gewichtszunahme (Average Daily Gain, ADG) eines Wiederkäuers; und/oder

- Verbessern der Futtereffizienz eines Wiederkäuers.

7. Verwendung nach Anspruch 5 oder 6, wobei die Zusammensetzung zwischen 20 und 40 Gew.-% Harnstoff, zwischen 20 und 40 Gew.-% Biuret und zwischen 21 und 37 Gew.-% einer oder mehrerer Nitratverbindungen, bezogen auf das Gesamtgewicht der Zusammensetzung, umfasst; wobei die Zusammensetzung insbesondere zwischen 25 und 35 Gew.-% Harnstoff, zwischen 25 und 35 Gew.-% Biuret und zwischen 23,5 und 31,5 Gew.-% einer oder mehrerer Nitratverbindungen, bezogen auf das Gesamtgewicht der Zusammensetzung, umfasst, wobei es sich bei der Nitratverbindung ganz insbesondere um ein wasserlösliches Nitratsalz handelt, ganz insbesondere ausgewählt aus Calciumnitrat, Magnesiumnitrat, Ammoniumnitrat, Kaliumnitrat und/oder Natriumnitrat.

8. Verwendung nach Anspruch 7, wobei die Zusammensetzung zwischen 20 und 32 Gew.-% Calciumnitrat und zwischen 1 und 5 Gew.-% Ammoniumnitrat, bezogen auf das Gesamtgewicht der Zusammensetzung, umfasst.

9. Verwendung nach einem der Ansprüche 5 bis 8, wobei die Zusammensetzung eine Wiederkäuerfutterergänzungszusammensetzung nach einem der Ansprüche 1 bis 4 ist.

10. Verfahren zum gleichzeitigen Reduzieren der enterischen Methanemissionen eines Wiederkäuers und zum Bereitstellen einer Nahrungs-Nichtproteinstickstoffquelle, umfassend das Verabreichen einer Wiederkäuerfutterergänzungszusammensetzung als NPN-Quelle an den Wiederkäuer, wobei die Wiederkäuerfutterergänzungszusammensetzung zwischen 5 und 60 Gew.-% Harnstoff, zwischen 2 und 60 Gew.-% Biuret und zwischen 20 und 40 Gew.-% einer oder mehrerer Nitratverbindungen, bezogen auf das Gesamtgewicht der Zusammensetzung, umfasst, und gegebenenfalls umfassend die Schritte des Herstellens oder Bereitstellens einer Wiederkäuerfutterzusammensetzung, die die Wiederkäuerfutterergänzungszusammensetzung als NPN-Quelle umfasst, vor der Verabreichung der Wiederkäuerfutterzusammensetzung an den Wiederkäuer.

11. Verfahren nach Anspruch 10, wobei die enterischen Methanemissionen des Wiederkäuers, ausgedrückt pro kg aufgenommener Trockenmasse oder pro kg aufgenommener organischer Substanz, um mindestens 5 % reduziert werden, wenn die Wiederkäuerfutterergänzungszusammensetzung einem Wiederkäuer als NPN-Quelle verabreicht wird, verglichen mit einer Ernährung, die Harnstoff als NPN-Quelle umfasst.

12. Verfahren nach Anspruch 10 oder 11, wobei das Verfahren weiterhin gleichzeitig ein Verfahren zum Verbessern der durchschnittlichen täglichen Gewichtszunahme (Average Daily Gain, ADG) eines Wiederkäuers ist, wobei die ADG des Wiederkäuers insbesondere um mindestens 5 %, ganz insbesondere um mindestens 10 % oder noch mehr insbesondere um mindestens 15 % erhöht wird, wenn die Wiederkäuerfutterergänzungszusammensetzung einem Wiederkäuer als NPN-Quelle verabreicht wird, im Vergleich zu einer Ernährung, die Harnstoff als NPN-Quelle umfasst, und wobei das Verfahren gegebenenfalls weiterhin gleichzeitig ein Verfahren ist zum

- Verbessern der Effizienz der mikrobiellen Proteinsynthese (EMPS) durch Pansenmikroben bei Wiederkäuern; und/oder

- Verringern des durch Pansenmikroben bei einem Wiederkäuer erzeugten Acetat-Propionat-Verhältnisses; und/oder

- Erhöhen der Verdaulichkeit der Trockenmasse (Dry Matter, DM) und der organischen Substanz (Organic Matter, OM); und/oder

- Verbessern der Futtereffizienz eines Wiederkäuers.

13. Verfahren nach einem der Ansprüche 10 bis 12, wobei die Wiederkäuerfutterergänzungszusammensetzung zwischen 20 und 40 Gew.-% Harnstoff, zwischen 20 und 40 Gew.-% Biuret und zwischen 21 und 37 Gew.-% einer oder mehrerer Nitratverbindungen, bezogen auf das Gesamtgewicht der Zusammensetzung, umfasst; wobei die Wiederkäuerfutterergänzungszusammensetzung als NPN-Quelle insbesondere zwischen 25 und 35 Gew.-% Harnstoff, zwischen 25 und 35 Gew.-% Biuret und zwischen 23,5 und 31,5 Gew.-% einer oder mehrerer Nitratverbindungen, bezogen auf das Gesamtgewicht der Zusammensetzung umfasst, wobei es sich bei der Nitratverbindung ganz insbesondere um ein wasserlösliches Nitratsalz handelt, wobei das wasserlösliche Nitratsalz noch mehr insbesondere ausgewählt ist aus Calciumnitrat, Magnesiumnitrat, Ammoniumnitrat, Kaliumnitrat und/oder Natriumnitrat, wobei die Wiederkäuerfutterergänzungszusammensetzung ganz besonders insbesondere zwischen 20 und 32 Gew.-% Calciumnitrat und zwischen 1 und 5 Gew.-% Ammoniumnitrat, bezogen auf das Gesamtgewicht der Zusammensetzung, umfasst.

14. Verfahren nach einem der Ansprüche 10 bis 13, wobei die Wiederkäuerfutterergänzungszusammensetzung weiterhin zwischen 1 und 5 Gew.-% Triuret, zwischen 0,5 und 3 Gew.-% Ammelid, zwischen 3 und 30 Gew.-% Cyanursäure und/oder zwischen 0,1 und 4,5 Gew.-% Feuchtigkeit umfasst, wobei Gew.-% auf das Gesamtgewicht der Zusammensetzung bezogen sind.

15. Verfahren nach einem der Ansprüche 10 bis 14, wobei die Wiederkäuerfutterergänzungszusammensetzung in der Wiederkäuerfutterzusammensetzung in einer Konzentration zwischen 0,5 und 4 Gew.-%, ganz insbesondere zwischen 0,5 und 3,5 Gew.-% oder zwischen 0,5 und 3 Gew.-%, noch mehr insbesondere zwischen 1 und 2,5 Gew.-%, vorliegt, wobei Gew.-% auf die Gesamttrockenmassebasis der Wiederkäuerfutterzusammensetzung bezogen sind.

**Revendications**

1. Composition de complément alimentaire pour ruminant comprenant entre 5 et 60 % en poids d'urée, entre 2 et 60 % en poids de biuret et entre 20 et 40 % en poids d'un ou plusieurs composés de nitrate, sur la base du poids total de la composition.

2. Composition de complément alimentaire pour ruminant selon la revendication 1, dans laquelle la composition comprend entre 20 et 40 % en poids d'urée, entre 20 et 40 % en poids de biuret, et entre 21 et 37 % en poids d'un ou plusieurs composés de nitrate, sur la base du poids total de la composition ; plus particulièrement dans laquelle la composition comprend entre 25 et 35 % en poids d'urée, entre 25 et 35 % en poids de biuret et entre 23,5 et 31,5 % en poids d'un ou plusieurs composés de nitrate, sur la base du poids total de la composition.

3. Composition de complément alimentaire pour ruminant selon la revendication 1 ou 2, dans laquelle le composé de nitrate est un sel de nitrate soluble dans l'eau, plus particulièrement choisi parmi le nitrate de calcium, le nitrate de magnésium, le nitrate d'ammonium, le nitrate de potassium et/ou le nitrate de sodium, et comprenant tout particulièrement entre 20 et 32 % en poids de nitrate de calcium et entre 1 et 5 % en poids de nitrate d'ammonium, sur la base du poids total de la composition.

4. Composition de complément alimentaire pour ruminant selon l'une quelconque des revendications 1 à 3, dans laquelle la composition comprend en outre entre 1 et 5 % en poids de triuret, entre 0,5 et 3 % en poids d'ammélide, entre 3 et 30 % en poids d'acide cyanurique, et/ou entre 0,1 et 4,5 % en poids d'humidité, les % en poids étant basés sur le poids total de la composition.

5. Utilisation d'une composition de complément alimentaire pour ruminant comprenant entre 5 et 60 % en poids d'urée, entre 2 et 60 % en poids de biuret et entre 20 et 40 % en poids d'un ou plusieurs composés de nitrate, sur la base du poids total de la composition, comme source d'azote non protéique alimentaire pour ruminant, et pour diminuer simultanément la production entérique de méthane d'un ruminant, et plus particulièrement comprenant en outre entre 1 et 5 % en poids de triuret, entre 0,5 et 3 % en poids d'ammélide, entre 3 et 30 % en poids d'acide cyanurique, et/ou entre 0,1 et 4,5 % en poids d'humidité, les % en poids étant basés sur le poids total de la composition.

6. Utilisation selon la revendication 5, pour en outre :

   - améliorer l'efficacité de la synthèse des protéines microbiennes (EMPS) par les microbes du rumen chez un ruminant ; et/ou
   - diminuer le rapport acétate/propionate produit par les microbes du rumen chez un ruminant ; et/ou
   - augmenter la digestibilité de la matière sèche (MS) et de la matière organique (OM) ; et/ou
   - pour améliorer le gain journalier moyen (ADG) d'un ruminant ; et/ou
   - pour améliorer l'efficacité alimentaire d'un ruminant.

**7.** Utilisation selon la revendication 5 ou 6, dans laquelle la composition comprend entre 20 et 40 % en poids d'urée, entre 20 et 40 % en poids de biuret, et entre 21 et 37 % en poids d'un ou plusieurs composés de nitrate, sur la base du poids total de la composition ; en particulier, dans laquelle la composition comprend entre 25 et 35 % en poids d'urée, entre 25 et 35 % en poids de biuret et entre 23,5 et 31,5 % en poids d'un ou plusieurs composés de nitrate, sur la base du poids total de la composition, plus particulièrement dans laquelle le composé de nitrate est un sel de nitrate soluble dans l'eau, et le plus particulièrement choisi parmi le nitrate de calcium, le nitrate de magnésium, le nitrate d'ammonium, le nitrate de potassium et/ou le nitrate de sodium.

**8.** Utilisation selon la revendication 7, dans laquelle la composition comprend entre 20 et 32 % en poids de nitrate de calcium et entre 1 et 5 % en poids de nitrate d'ammonium, sur la base du poids total de la composition.

**9.** Utilisation selon l'une quelconque des revendications 5 à 8, dans laquelle la composition est une composition de complément alimentaire pour ruminant selon l'une quelconque des revendications 1 à 4.

**10.** Procédé permettant de réduire simultanément les émissions entériques de méthane d'un ruminant et de fournir une source d'azote non protéique alimentaire, comprenant l'administration au ruminant d'une composition de complément alimentaire pour ruminant en tant que source de NPN, ladite composition de complément alimentaire pour ruminant comprenant entre 5 et 60 % en poids d'urée, entre 2 et 60 % en poids de biuret et entre 20 et 40 % en poids d'un ou plusieurs composés de nitrate, sur la base du poids total de la composition, et comprenant éventuellement les étapes de préparation ou de fourniture d'une composition alimentaire pour ruminant comprenant la composition de complément alimentaire pour ruminant en tant que source de NPN avant l'administration de la composition alimentaire pour ruminant au ruminant.

**11.** Procédé selon la revendication 10, dans lequel les émissions entériques de méthane du ruminant, exprimées par kg d'assimilation de matière sèche ou par kg d'assimilation de matière organique, sont réduites d'au moins 5 % lorsque la composition de complément alimentaire pour ruminant est administrée à un ruminant en tant que source de NPN par rapport à un régime alimentaire comprenant de l'urée en tant que source de NPN.

**12.** Procédé selon la revendication 10 ou 11, dans lequel ledit procédé est en outre simultanément un procédé pour améliorer le gain journalier moyen (ADG) d'un ruminant, en particulier dans lequel l'ADG du ruminant est augmenté d'au moins 5 %, plus particulièrement d'au moins 10 %, ou encore plus particulièrement d'au moins 15 %, lorsque la composition de complément alimentaire pour ruminant est administrée à un ruminant en tant que source de NPN par rapport à un régime alimentaire comprenant de l'urée en tant que source de NPN, et, éventuellement, dans lequel ledit procédé est en outre simultanément un procédé pour

- améliorer l'efficacité de la synthèse des protéines microbiennes (EMPS) par les microbes du rumen chez un ruminant ; et/ou
- diminuer le rapport acétate/propionate produit par les microbes du rumen chez un ruminant ; et/ou
- augmenter la digestibilité de la matière sèche (MS) et de la matière organique (OM) ; et/ou
- améliorer l'efficacité alimentaire d'un ruminant.

**13.** Procédé selon l'une quelconque des revendications 10 à 12, dans lequel la composition de complément alimentaire pour ruminant comprend entre 20 et 40 % en poids d'urée, entre 20 et 40 % en poids de biuret, et entre 21 et 37 % en poids d'un ou plusieurs composés de nitrate, sur la base du poids total de la composition ; en particulier, dans lequel la composition de complément alimentaire pour ruminant en tant que source de NPN comprend entre 25 et 35 % en poids d'urée, entre 25 et 35 % en poids de biuret et entre 23,5 et 31,5 % en poids d'un ou plusieurs composés de nitrate, sur la base du poids total de la composition, plus particulièrement dans lequel le composé de nitrate est un sel de nitrate soluble dans l'eau, encore plus particulièrement dans lequel le sel de nitrate soluble dans l'eau est choisi parmi le nitrate de calcium, le nitrate de magnésium, le nitrate d'ammonium, le nitrate de potassium et/ou le nitrate de sodium, dans lequel le plus particulièrement la composition de complément alimentaire pour ruminant comprend entre 20 et 32 % en poids de nitrate de calcium et entre 1 et 5 % en poids de nitrate d'ammonium, sur la base du poids total de la composition.

**14.** Procédé selon l'une quelconque des revendications 10 à 13, dans lequel la composition de complément alimentaire pour ruminant comprend en outre entre 1 et 5 % en poids de triuret, entre 0,5 et 3 % en poids d'ammélide, entre 3 et 30 % en poids d'acide cyanurique et/ou entre 0,1 et 4,5 % en poids d'humidité, les % en poids étant basés sur le poids total de la composition.

**15.** Procédé selon l'une quelconque des revendications 10 à 14, dans lequel la composition de complément alimentaire pour ruminant est présente dans la composition alimentaire pour ruminant en une concentration comprise entre 0,5 et 4 % en poids, plus particulièrement entre 0,5 et 3,5 % en poids ou entre 0,5 et 3 % en poids, encore plus particulièrement entre 1 et 2,5 % en poids, les % en poids étant basés sur la matière sèche totale de la composition alimentaire pour ruminant.

**FIG. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017181250 A

- US 2768895 A

**Non-patent literature cited in the description**

- **XIUMIN ZHANG et al.** Effects of urea plus nitrate pretreated rice straw and corn oil supplementation on fiber digestibility, nitrogen balance, rumen fermentation, microbiota and methane emissions in goats. *Journal of Animal Science and Biotechnology, Biomed, Central Ltd.*, 23 January 2019, vol. 10 (1), 1-10

- Effect of dietary nitrate on enteric methane emissions, production performance and rumen fermentation of dairy cows grazing kikuyu-dominant pasture during summer. **VAN WYNGAARD J.D.V. et al.** Animal Feed Science and Technology. Elsevier, 13 August 2018, vol. 244, 76-87
- **VAN SOEST et al.** Ankom 200 Fiber Analyzer. Ankom Technology Corp., 1991